# EUROPEAN PATENT SPECIFICATION

(11) **EP 2 684 524 B1**
(45) Date of publication and mention of the grant of the patent: **04.01.2017**
(21) Application number: 12755405.3
(22) Date of filing: 08.03.2012
(51) Int. Cl.: A61B 8/00, A61B 8/14, A61B 8/08, A61B 5/00, G01N 29/24

(54) **TOMOGRAPHIC IMAGE GENERATION DEVICE AND METHOD**
TOMOGRAPHISCHE BILDERZEUGUNGSVORRICHTUNG UND VERFAHREN DAFÜR
DISPOSITIF ET PROCÉDÉ DE GÉNÉRATION D'IMAGE TOMOGRAPHIQUE

(30) Priority: 10.03.2011 JP 2011052743; 18.01.2012 JP 2012007610
(43) Date of publication of application: 15.01.2014
(73) Proprietor: FUJIFILM Corporation, Tokyo 106-8620 (JP)
(72) Inventor: HIROTA, Kazuhiro, Ashigarakami-gun Kanagawa 258-8538 (JP)
(74) Representative: Klunker . Schmitt-Nilson . Hirsch
(86) International application number: PCT/JP2012/001598
(87) International publication number: WO 2012/120889

(56) References cited:
- EP-A1- 1 561 424
- WO-A1-2011/102105
- JP-A- 2005 218 684
- JP-A- 2009 540 904
- US-A1- 2008 071 172
- US-A1- 2009 187 099
- US-A1- 2011 319 743
- Anonymous: "Data buffer - Wikipedia, the free encyclopedia", , 2 March 2011 (2011-03-02), XP055129600, Retrieved from the Internet: URL:http://en.wikipedia.org/w/index.php?ti tle=Data_buffer&oldid=416747643 [retrieved on 2014-07-17]

## Description

### Technical Field

The present invention is related to a tomographic image generating apparatus according to the preamble of claim 1 and a tomographic image generating method. More specifically, the present invention is related to a tomographic image generating apparatus that generate ultrasound images based on reflected ultrasonic waves and generates photoacoustic images based on photoacoustic signals.

### Background Art

The ultrasound examination method is known as an image examination method that enables examination of the state of the interior of living organisms in a non invasive manner. Ultrasound examination employs an ultrasound probe capable of transmitting and receiving ultrasonic waves. When the ultrasonic waves are transmitted to a subject (living organism) from the ultrasound probe, the ultrasonic waves propagate through the interior of the living organisms, and are reflected at interfaces among tissue systems. The ultrasound probe receives the reflected ultrasonic waves and images the state of the interior of the subject, by calculating distances based on the amounts of time that the reflected ultrasonic waves return to the ultrasound probe.

Photoacoustic imaging, which images the interiors of living organisms utilizing the photoacoustic effect, is also known. Generally, in photoacoustic imaging, pulsed laser beams are irradiated into living organisms. Biological tissue within the living organisms that absorbs the energy of the pulsed laser beams generates ultrasonic waves (photoacoustic signals) by adiabatic expansion thereof. An ultrasound probe or the like detects the photoacoustic signals, and constructs photoacoustic images based on the detected signals, to enable to enable visualization of the living organisms based on the photoacoustic signals.

An apparatus capable of generating both photoacoustic images and ultrasound images is disclosed in Patent Document 1, for example. The biological data imaging apparatus disclosed in Patent Document 1 irradiates a laser beam onto a subject and receives photoacoustic signals generated due to laser irradiation when a command to initiate collection of photoacoustic image data is input. The biological data imaging apparatus generates a photoacoustic image based on the received signals, and stores the generated photoacoustic image in an image data memory A for photoacoustic images. After the photoacoustic image is generated, the biological data imaging apparatus transmits and receives ultrasonic waves when a command to initiate collection of ultrasound image data is input, and generates an ultrasound image based on the received ultrasonic waves. The biological data imaging apparatus stores the generated ultrasound image in an image data memory B for ultrasound images.

### [Prior Art Documents]

### [Patent Documents]

[Patent Document 1]
Japanese Unexamined Patent Publication No. 2010-012295

US 2009/187099 A1 discloses a tomographic image generating apparatus having the features included in the preamble of claim 1. This prior art tomographic image generating apparatus as means to make light trigger signals and ultrasound transmission trigger signals having a certain delay time there between. Separate discrete buffer memories are provided for storing photoacoustic signals and ultrasound signals.

EP 1 561 424 A1 discloses a tomographic image generating apparatus, in which a laser light source is adapted to output different laser light beams having different wave lenghts.

### Disclosure of the Invention

Generally, in an apparatus capable of generating photoacoustic images and ultrasound images, photoacoustic images and ultrasound images are stored in separate memories, as in Patent Document 1. For example, in the case that received photoacoustic signals and ultrasonic wave signals are stored in memories prior to image construction, a memory for storing photoacoustic signals and a memory for storing ultrasonic wave signals are provided separately, and the two types of signals are stored in the separate memories. In this case, it is necessary to switch memories after reception of the photoacoustic signals is completed considering the fact that reception of ultrasonic wave signals is performed following reception of photoacoustic signals. Therefore, reception of the ultrasonic wave signals cannot be performed until switching of the memories is completed. In such a case, waste of time occurs for the amount of time required to switch the memories, and this wasted time prevented acceleration of processing time.

The present invention has been developed in view of the foregoing circumstances. It is an object of the present invention to provide a tomographic image generating apparatus and a tomographic image generating method in which the amount of time required until reception of photoacoustic signals and ultrasonic wave signals is completed is shortened, thereby expediting processing speed.

In order to achieve the above object, the present invention provides a tomographic image generating apparatus, comprising the features of claim 1.

Preferred embodiments are defined by the independent claims.

the ultrasound image generating means comprises a 1/2 resampling means that resamples the sampled reflected acoustic signals to 1/2, and generates the ultrasound images based on the 1/2 resampled reflected acoustic signals.

The tomographic image generating apparatus of the present invention may adopt a configuration, wherein:
the 1/2 resampling means compresses the sampled reflected acoustic waves to 1/2 in the direction of a temporal axis.

As an alternative to the above, the tomographic image generating apparatus of the present invention may further comprise:
sampling rate control means for controlling the sampling rate of the sampling means; and wherein:
   the sampling rate control means controls the sampling rate when the sampling means samples the reflected acoustic signals to be half the sampling rate of the sampling rate when sampling the photoacoustic signals. In this case, a configuration may be adopted, wherein:
      the sampling rate control means controls the sampling rate synchronized with the ultrasonic wave trigger signal or with light irradiation onto the subject.

The trigger control section may output the ultrasonic wave trigger signal following output of the light trigger signal. Alternatively, the trigger control section may output the light trigger signal following output of the ultrasonic wave trigger signal.

The tomographic image generating apparatus of the present invention may adopt a configuration, wherein:
a range onto which ultrasonic waves are transmitted from the ultrasonic wave transmitting section and a range in which the acoustic signal detecting section detects the photoacoustic signals and the reflected acoustic signals are each divided into a plurality of regions; and
the trigger control section outputs the light trigger

A configuration may be adopted, wherein the wavelength selecting element includes a plurality of band pass filters that transmit wavelengths different from each other, and the light source unit further has a drive means that drives the wavelength selecting element such that the band pass filter which is inserted into the optical path of the optical resonator is sequentially switched in a predetermined order.

The wavelength selecting element may be constituted by a rotatable filter body that switches band pass filters which are selectively inserted into the optical path of the optical resonator accompanying rotational displacement, and a drive means that rotationally drives the rotatable filter body.

A configuration may be adopted, wherein:
the image generating means comprises a two wavelength calculating means that extracts the relationships among signal intensities for each of the laser beams of different wavelengths irradiated onto the subject and received by the probe; and
the photoacoustic images are generated based on the relationships among the signal intensities extracted by the 2 wavelength calculating means.

A configuration may be adopted, wherein:
the image generating means further comprises intensity data extracting means for generating intensity data that represents signal intensities based on photoacoustic signals corresponding to each of the plurality of wavelengths, determines the gradation value of each pixel within the photoacoustic images based on the intensity data, and determines the color that each pixel is displayed in based on the relationship among signal intensities.

A configuration may be adopted, wherein:
the plurality of wavelengths of pulsed laser beams output by the light source includes a first wavelength and a second wavelength; and
the image generating means further comprises complexifying means for generating complex number data, in which one of photoacoustic signals received by the probe when the pulsed laser beam of the first wavelength is irradiated onto the subject and photoacoustic signals received by the probe when the pulsed laser beam of the second wavelength is irradiated onto the subject is designated as an real part and the other is designated as a imaginary part, and reconstructing means for generating reconstructed images from the complex number data by Fourier transform, wherein an intensity ratio extracting means extracts phase data as the relationship among signal intensities from the reconstructed images, and an intensity data extracting means extracts intensity data from the reconstructed images.

In the tomographic image generating apparatus of the present invention, acoustic signal detecting elements of the acoustic signal detecting section may also function as ultrasonic transmission elements of the ultrasonic wave transmitting section.

The present invention also provides a tomographic image generating method, comprising the steps of claim 14.

The tomographic image generating apparatus and the tomographic image generating method of the present invention executes one of light irradiation onto a subject and ultrasonic wave transmission toward the subject, initiates sampling of photoacoustic signals or reflected acoustic signals, executes the other of light irradiation and ultrasonic wave transmission while maintaining the sampling state, to sample reflected acoustic signals or photoacoustic signals, and stores the sampled photoacoustic signals and the sampled reflected acoustic signals into a common memory. IN the present invention, sampling is not interrupted nor the memory switched when transitioning from sampling of one of the photoacoustic signals and the reflected acoustic signals to the other of the photoacoustic signals and the reflected acoustic signals. Therefore, the time required until completion of reception of the photoacoustic signals the ultrasonic wave signals can be shortened, and processing can be accelerated.

### Brief Description of the Drawings

Figure 1 is a block diagram that illustrates a tomographic image generating apparatus according to a first embodiment of the present invention.
Figure 2 is a flow chart that illustrates the operational procedures of the tomographic image generating apparatus of the first embodiment.
Figure 3 is a diagram that illustrates an example of division into blocks.
Figure 4A is a diagram that illustrates light irradiation within an area A.
Figure 4B is a diagram that illustrates detection of photoacoustic signals within the area A.
Figure 4C is a diagram that illustrates ultrasonic wave transmission within the area A.
Figure 4D is a diagram that illustrates detection of reflected acoustic signals within the area A.
Figure 5 is a timing chart that illustrates a timing chart of an exemplary an operation the first embodiment.
Figure 6 is a block diagram that illustrates a tomographic image generating apparatus according to a second embodiment of the present invention.
Figure 7 is a flow chart that illustrates the operational procedures of the tomographic image generating apparatus of the second embodiment.
Figure 8 is a timing chart that illustrates a timing chart of an exemplary operation of the second embodiment.
Figure 9 is a block diagram that illustrates a tomographic image generating apparatus according to a third embodiment of the present invention.
Figure 10 is a block diagram that illustrates a variable wavelength laser unit.
Figure 11 is a diagram that illustrates an example of the configurations of a wavelength selecting element, a drive means, and a driving state detecting means.
Figure 12 is a timing chart that illustrates a timing chart of an exemplary operation of the third embodiment.
Figure 13 is a timing chart that illustrates a timing chart of an exemplary operation of a modification of the present invention.
Figure 14 is a diagram that illustrates an example in which a light irradiating section is provided at a position facing a probe.
Figure 15 is a diagram that illustrates a state in which light is irradiated from an facing position.

### Best Mode for Carrying out the Invention

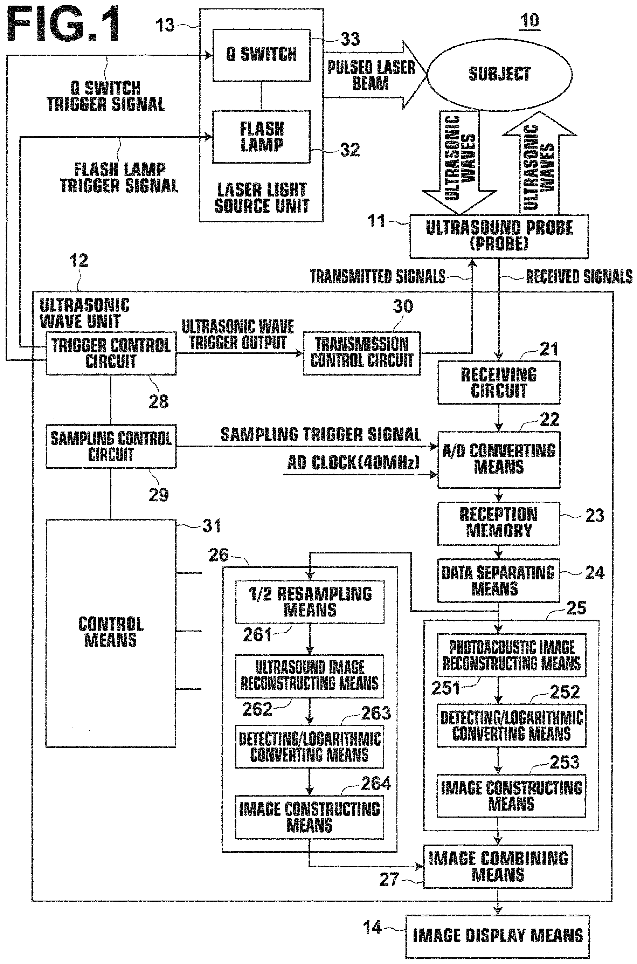

Hereinafter, embodiments of the present invention will be described in detail with reference to the attached drawings. Figure 1 illustrates a tomographic image generating apparatus 10 according to a first embodiment of the present invention. The tomographic image generating apparatus 10 includes: an ultrasound probe (probe) 11; an ultrasonic wave unit 12; and a light source (laser unit) 13. The laser unit 13 is a light source, and generates a laser beam to be irradiated onto subject. The wavelength of the light beam to be irradiated onto subjects may be set as appropriate according to targets of observation. The laser beam output by the laser unit 13 is guided to the probe 11 by a light guiding means such as an optical fiber, then irradiated onto subjects from the probe 11.

The probe 11 includes an ultrasonic wave transmitting section that outputs (transmits) ultrasonic waves to subjects and an ultrasonic wave detecting section (acoustic signal detecting section) that detects (receives) acoustic waves reflected by the subjects. Here, ultrasonic wave detecting elements of the ultrasonic wave detecting section may also function as ultrasonic wave transmitting elements of the ultrasonic wave transmitting section. An ultrasonic transducer (element) may be employed to transmit and detect ultrasonic waves. The probe 11 has a plurality of ultrasonic transducers which are arranged one dimensionally, for example. The probe outputs ultrasonic waves from the plurality of ultrasonic transducers when generating ultrasound images and detects reflected ultrasonic waves (hereinafter, also referred to as "reflected acoustic signals") with the plurality of ultrasonic transducers. The probe 11 detects ultrasonic waves (hereinafter, also referred to as "photoacoustic signals") which are generated by targets of measurement within subjects absorbing the laser beam output by the laser unit 13 when generating photoacoustic images. Note that it is not necessary for the probe 11 to include both the ultrasonic wave transmitting section and the ultrasonic wave detecting section at least. The ultrasonic wave transmitting section and the ultrasonic wave detecting section may be separated, and transmission of ultrasonic waves and reception of ultrasonic waves may be performed at different locations.

The ultrasonic wave unit 12 has a receiving circuit 21, an A/D converting means 22, a reception memory 23, a data separating means 24, photoacoustic image generating means 25, an ultrasound image generating means 26, an image combining means 27, a trigger control circuit 28, a sampling control circuit 29, a transmission control circuit 30, and a control means 31. The control means 31 controls each component of the ultrasonic wave unit 12. The receiving circuit 21 receives ultrasonic waves (photoacoustic signals or reflected acoustic signals) detected by the plurality of ultrasonic transducers of the probe 11. The A/D converting means 22 is a sampling means, and converts the ultrasonic wave signals received by the receiving circuit 21 into digital signals. The A/D converting means 22 samples the ultrasonic signals at a predetermined sampling period synchronized with an A/D clock signal, for example.

The trigger control circuit 28 is a trigger control means, and outputs a light trigger signal that commands light output to the laser unit 13. The laser unit 13 includes a flash lamp 32 that pumps a laser medium (not shown) such as YAG or titanium sapphire, and a Q switch 33 that controls laser oscillation. When the trigger control circuit 28 outputs a flash lamp trigger signal, the laser unit 13 lights the flash lamp 32 and pumps the laser medium. The trigger control circuit 28 outputs a Q switch trigger signal after the flash lamp 32 sufficiently pumps the laser medium, for example. The Q switch is turned ON when the Q switch trigger signal is received, and causes a laser beam to be output from the laser unit 13. The amount of time required from the timing that the flash lamp 32 is lit to a point in time at which the laser medium is sufficiently pumped can be estimated from the properties of the laser medium. The Q switch 33 may be turned ON within the laser unit 13 instead of the Q switch being controlled by the trigger control circuit 28. In this case, a signal that indicates that a Q switch has been turned ON may be transmitted to the ultrasonic wave unit 12 from the laser unit 13. Here, the light trigger signal is a concept that includes at least one of the flash lamp trigger signal and the Q switch trigger signal. The Q switch trigger signal corresponds to the light trigger signal in the case that the trigger control circuit 28 outputs the Q switch trigger signal. The flash lamp trigger signal corresponds to the light trigger signal in the case that the laser unit 13 generates the timing of the Q switch trigger.

In addition, the trigger control circuit 28 outputs an ultrasonic wave trigger signal that commands ultrasonic wave transmission to the transmission control circuit 30. When the trigger signal is received, the transmission control circuit 30 causes the probe 11 to transmit ultrasonic waves. The trigger control circuit 28 outputs a light trigger signal first, and then outputs an ultrasonic wave trigger signal, for example. Irradiation of a laser beam and detection of photoacoustic signals are performed by the light trigger signal being output, and transmission of ultrasonic waves toward a subject and detection of reflected acoustic signals are performed thereafter by output of the ultrasonic wave trigger signal.

The sampling control circuit 29 outputs a sampling trigger signal that commands initiation of sampling to the A/D converting means 22. the sampling control circuit 29 outputs a sampling trigger signal at a timing following output of a light trigger signal by the trigger control circuit 28 and prior to output of an ultrasonic wave trigger signal. The sampling control circuit 29 outputs a sampling trigger signal at a timing following output of the light trigger signal, and preferably at a timing at which a laser beam is actually irradiated onto a subject. For example, the sampling control circuit 29 outputs a sampling trigger signal synchronized with the timing at which the trigger control circuit 28 outputs a Q switch trigger signal. When the sampling trigger signal is received, the A/D converting means 22 initiates sampling of ultrasonic waves (photoacoustic signals) detected by the probe 11.

Following output of the light trigger signal, the trigger control circuit 28 outputs an ultrasonic wave trigger signal at a timing that detection of photoacoustic signals is completed. At this time, the A/D converting means 22 does not interrupt sampling of ultrasonic wave signals, but continues to execute sampling. In other words, the trigger control circuit 28 outputs the ultrasonic wave trigger signal in a state in which the A/D converting means 22 is continuing sampling of the ultrasonic wave signals. The ultrasonic waves detected by the probe 11 change from photoacoustic waves to reflected acoustic waves, by the probe 11 transmitting ultrasonic waves in response to the ultrasonic wave trigger signal. The A/D converting means 22 continuously samples the photoacoustic waves and the reflected acoustic waves, by continuing sampling of detected ultrasonic wave signals.

The A/D converting means 22 stores both the sampled photoacoustic signals and the sampled reflected acoustic signals in the common reception memory 23. A semiconductor memory device, for example, may be employed as the reception memory. Other memory devices, such as a magnetic memory device, may also be employed as the reception memory 23. The sampled data stored in the reception memory 23 are data of photoacoustic signals up to a certain point in time, and become data of reflected acoustic signals after the point in time. The data separating means 24 separates the photoacoustic signals and the ultrasonic wave signals stored in the reception memory 23. The data separating means 24 provides the separated photoacoustic signals to the photoacoustic image generating means 25, and provides the separated ultrasonic wave signals to the ultrasound image generating means 26.

The photoacoustic image generating means 25 generates photoacoustic images based on photoacoustic signals. The photoacoustic image generating means 25 includes: a photoacoustic image reconstructing means 251; a detecting/logarithmic converting means 252; and a photoacoustic image constructing means 253. The ultrasound image generating means generates ultrasound images based on reflected acoustic signals. The ultrasound image generating means 26 includes: a 1/2 resampling means 261; an ultrasound image reconstructing means 262; a detecting/logarithmic converting means 263; and an ultrasound image constructing means 264. The functions of each component of the photoacoustic image generating means 25 and the ultrasound image generating means 26 may be realized by a computer executing processes according to predetermined programs.

The photoacoustic image reconstructing means 251 receives photoacoustic signals from the data separating means 24. The photoacoustic image reconstructing means 251 generates data corresponding to each line of photoacoustic images, which are tomographic images, based on the photoacoustic signals. The image reconstructing means 18 adds data from 64 ultrasonic transducers of the probe 11 at delay times corresponding to the positions of the ultrasonic transducers, to generate data corresponding to a single line (delayed addition method), for example. Alternatively, the photoacoustic image reconstructing means 251 may execute image reconstruction by the CBP (Circular Back Projection) method. As further alternatives, the photoacoustic image reconstructing means 251 may execute image reconstruction by the Hough transform method or Fourier transform method.

The detecting/logarithmic converting means 252 generates envelope curves of data that represent each line output by the photoacoustic image reconstructing means 251, and logarithmically converts the envelope curves to widen the dynamic ranges thereof. The photoacoustic image constructing means 253 generates photoacoustic images based on data that represent each line, on which logarithmic conversion has been administered. The photoacoustic image constructing means 253 generates photoacoustic images by converting the positions of photoacoustic signals (peak portions) along a temporal axis to positions in the depth direction of the photoacoustic images, for example.

The 1/2 resampling means 261 receives reflected acoustic signals from the data separating means 24, and resamples the reflected acoustic signals to 1/2. The 1/2 resampling means 261 compresses the reflected acoustic signals into 1/2 in the direction of a temporal axis, for example. The reason why resampling is performed is as follows. If photoacoustic waves and reflected acoustic waves are generated at the same position in the depth direction of a subject, time is necessary for ultrasonic waves transmitted from the probe 11 to propagate to this position in the case of reflected acoustic waves. Therefore, the amount of time from ultrasonic wave transmission to reflected acoustic wave detection will be double the amount of time from light irradiation to photoacoustic wave detection. That is, photoacoustic signals can be detected within an amount of time required for a one way trip, whereas reflected signals require an amount of time for a round trip.

The ultrasound image reconstructing means 262 generates data for each line of ultrasound images, which are tomographic images, based on resampled ultrasonic wave signals. The detecting/logarithmic converting means 263 generates envelope curves of data that represent each line output by the ultrasound image reconstructing means 262, and logarithmically converts the envelope curves to widen the dynamic ranges thereof. The ultrasound image constructing means 264 generates photoacoustic images based on data that represent each line, on which logarithmic conversion has been administered. The ultrasound image generating means 26 may generate ultrasound images in the same manner as that in which the photoacoustic image generating means 25 generates photoacoustic images, except that the signals are 1/2 resampled ultrasonic wave signals.

The image combining means 27 combines the photoacoustic images generated by the photoacoustic image generating means 25 and the ultrasound image generated by an ultrasound image generating means 26. The image combining means 27 combines the images by overlapping the photoacoustic images on the ultrasound images, for example. The images combined by the image combining means 27 are displayed on a display monitor or the like by an image display means 14. It is also possible for the image display means 14 to display only one of the photoacoustic images and the ultrasound image, or to display the photoacoustic image and the ultrasound image arranged next to each other, without combining the images.

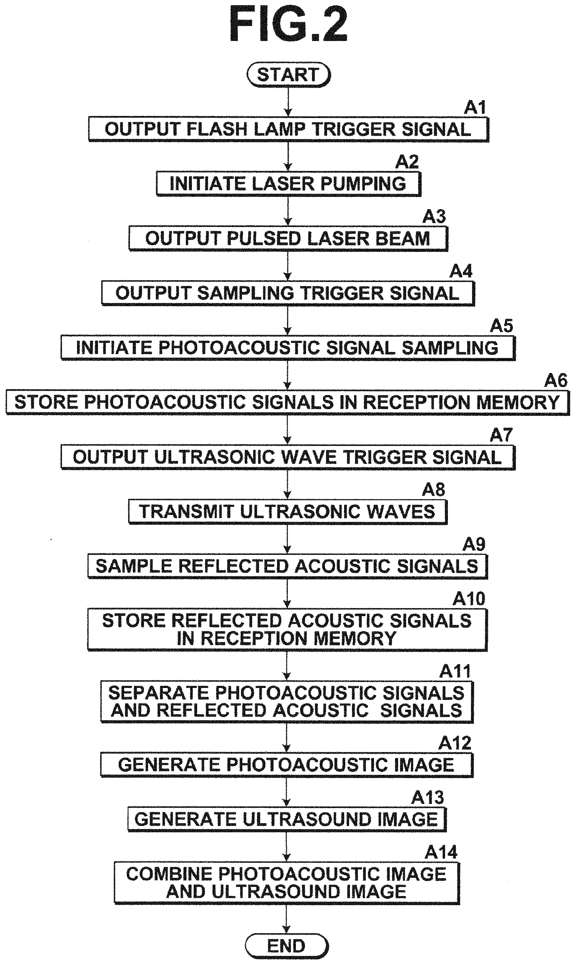

Figure 2 illustrates operational procedures. The trigger control circuit 28 outputs a flash lamp trigger signal to the laser unit 13 (step A1). The flash lamp 32 of the laser unit 13 is lit in response to the flash lamp trigger signal, and pumping of the laser medium is initiated (step A2). The trigger control circuit 28 outputs a Q switch trigger signal to the laser unit 13 to turn the Q switch ON, thereby causing a pulsed laser bema to be output from the laser unit 13 (step A3). The trigger control circuit 28 outputs the Q switch trigger signal at a timing having a predetermined temporal relationship with the timing at which the flash lamp trigger signal is output. The laser beam output from the laser unit 13 is irradiated onto a subject. Photoacoustic signals are generated within the subject due to the irradiated pulsed laser beam. The probe 11 detects the photoacoustic signals generated within the subject.

The sampling control circuit 29 sends a sampling trigger signal to the A/D converting means 22 synchronized with the timing at which the laser is output (step A4). The sampling control circuit 29 outputs the sampling trigger signal at the same timing as the timing at which the trigger control circuit 28 outputs the Q switch trigger signal, for example. The photoacoustic signals detected by the probe 11 are input to the A/D converting means 22 via the receiving circuit 21, and the A/D converting means 22 initiates sampling of the photoacoustic signals (step A5). The A/D converting means 22 samples the photoacoustic signals at a sampling rate of 40M (samples)/second, based on an A/D clock signal having a clock frequency of 40MHz. The A/D converting means 22 stores the sampled photoacoustic signals in the reception memory 23 (step A6).

The trigger control circuit 28 outputs an ultrasonic wave trigger signal at a predetermined timing (step A7). The trigger control circuit 28 outputs the ultrasonic wave trigger signal at a timing at which the number of pieces of sampled data sampled from initiation of sampling by the A/D converting means 22 reaches a predetermined number. In other words, the trigger control circuit 28 outputs the ultrasonic wave trigger signal at a timing after a predetermined amount of time has elapsed form the timing that the sampling control circuit 29 outputs the sampling trigger signal. At this time, the A/D converting means 22 does not interrupt sampling, and maintains a sampling state.

When the ultrasonic wave trigger signal that commands transmission of ultrasonic waves is received via the transmission control circuit 30, the probe 11 transmits ultrasonic waves toward the subject (step A8). The probe 11 detects reflected acoustic signals of the transmitted ultrasonic waves after transmitting the ultrasonic waves. The A/D converting means 22 samples the reflected acoustic signals continuous with sampling of the photoacoustic signals (step A9). The sampling rate for the reflected acoustic signals is the same as the sampling rate when sampling the photoacoustic signals. The A/D converting means 22 stores the sampled reflected acoustic signals in the reception memory 23 continuous with the stored photoacoustic signals. Here, it is only necessary for the photoacoustic signals and the reflected acoustic signals to be theoretically continuously recorded, and it is not necessary for the photoacoustic signals and the reflected acoustic signals to be physically continuously recorded. For example, in the case that the reception memory 23 includes a plurality of semiconductor memory chips, and a single theoretical memory is constituted by the plurality of semiconductor memory chips, the photoacoustic signals and the reflected acoustic signals may be recorded across a plurality of chips. Sampling is completed when the A/D converting means 22 samples a predetermined number of reflected acoustic signals.

The data separating means 24 separates the photoacoustic signals and the ultrasonic wave signals from the reception memory 23, in which the photoacoustic signals and the ultrasonic wave signals are continuously stored, provides the photoacoustic signals to the photoacoustic image generating means 25, and provides the ultrasonic wave signals to the ultrasound image generating means 26 (step A11). The photoacoustic image generating means 25 generates a photoacoustic image based on the photoacoustic signals (step A12). The ultrasound image generating means 26 resamples the ultrasonic wave signals to 1/2, and generates an ultrasound image (step A13). The image combining means 27 combines the photoacoustic image and the ultrasound image (step A14), and displays the combined image on the display screen of the image display means 14.

Detection of the photoacoustic signals and transmission and reception of the ultrasonic waves may be performed within the entire range of ultrasonic transducers of the probe 11. Alternatively, the range in which detection of the photoacoustic signals and transmission and reception of the ultrasonic waves is performed may be divided into a plurality of regions (blocks), and the detection, transmission, and reception may be performed separately for each region. In the case that the range is divided into the regions and detection of the photoacoustic signals and transmission and reception of the ultrasonic waves are performed for each region, output of a light trigger signal, output of a sampling trigger signal, and output of an ultrasonic wave trigger signal are performed for each region. When sampling of photoacoustic signals and reflected acoustic signals is completed for one region, the above procedures may be performed within a next region.

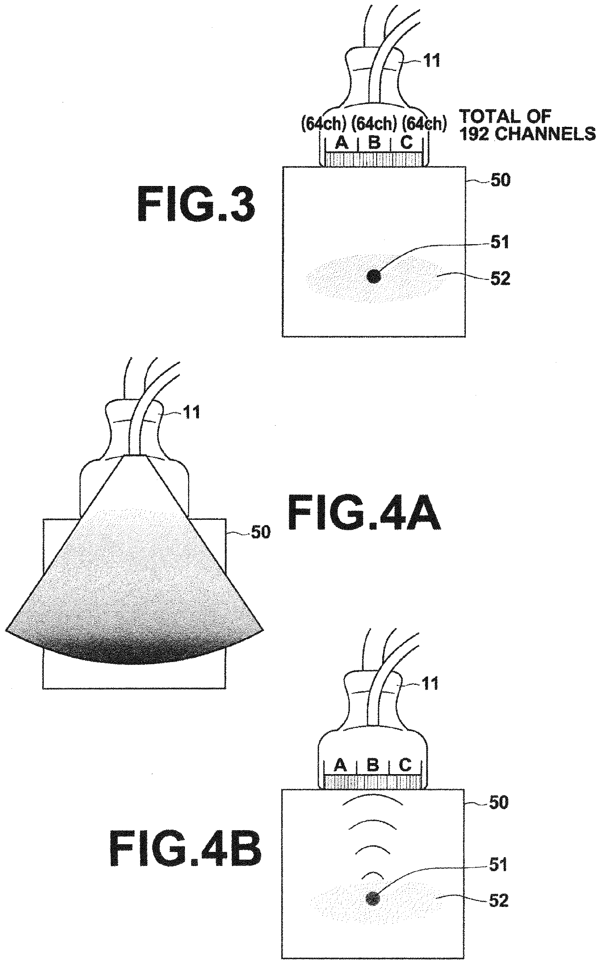

Figure 3 illustrates an example of division into blocks. Assume, for example, that the probe 11 has 192 ultrasonic transducers. A subject 50 has a light absorber 51 that generates photoacoustic waves due to irradiation of a pulsed laser beam, and a reflector 52 that reflects ultrasonic waves, directly under the probe 11. The range of all elements is divided into three areas, Area A, Area B, and Area C, by dividing the 192 ultrasonic transducers into regions having 64 elements each, for example. In this case, irradiation of light, detection of photoacoustic signals, transmission of ultrasonic waves, and detection of reflected acoustic signals may be executed in each of the three areas.

Figure 4A through Figure 4D illustrate irradiation of light, detection of photoacoustic signals, transmission of ultrasonic waves, and detection of reflected acoustic signals in Area A. First, at step A2 of Figure 2, a pulsed laser beam is irradiated onto the entire region of the subject 50 that includes Area A (Figure 4A). The light absorber 51 absorbs the energy of the pulsed laser beam, and photoacoustic signals are generated at the position of the light absorber 51 due to adiabatic expansion thereof. The probe 11 detects the photoacoustic signals with the 64 elements corresponding to Area A from among the 192 elements (Figure 4B). The remaining ultrasonic transducers corresponding to Area B and Area C are placed in a standby state. The A/D converting means 22 (Figure 1) samples the photoacoustic signals detected by the 64 elements corresponding to Area A at step A5, and stores the sampled photoacoustic signals in the reception memory 23 at step A6.

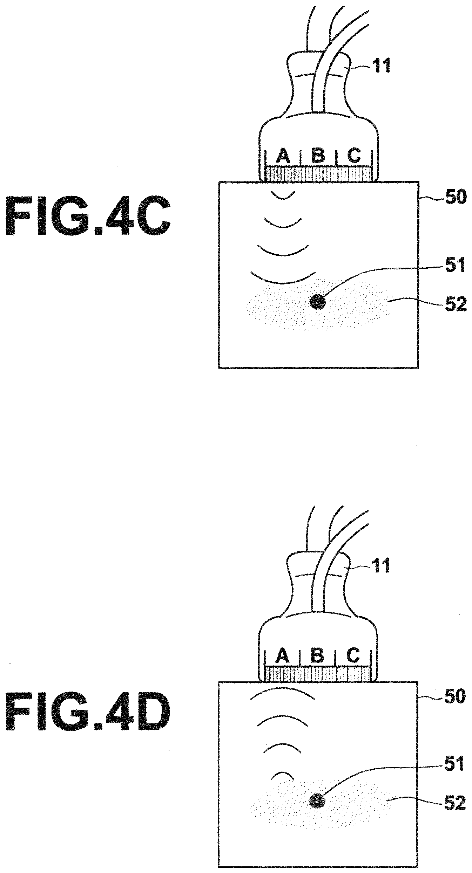

Next, ultrasonic waves are transmitted from the 64 elements that correspond to Area A from among the 192 elements of the probe 11, at step A8 (Figure 4C). The transmitted ultrasonic waves are reflected by the reflector 52, and reflected acoustic signals are generated. The probe 11 detects the reflected acoustic signals with the 64 elements corresponding to Area A from among the 192 elements (Figure 4D). The A/D converting means 22 samples the reflected acoustic signals detected by the 64 elements corresponding to Area A at step A9, and stores the sampled photoacoustic signals in the reception memory 23 at step A10. With respect to Area B and Area C, ultrasonic transducers that correspond to each area are employed to detect photoacoustic signals, transmit ultrasonic waves, and receive ultrasonic waves in a similar manner.

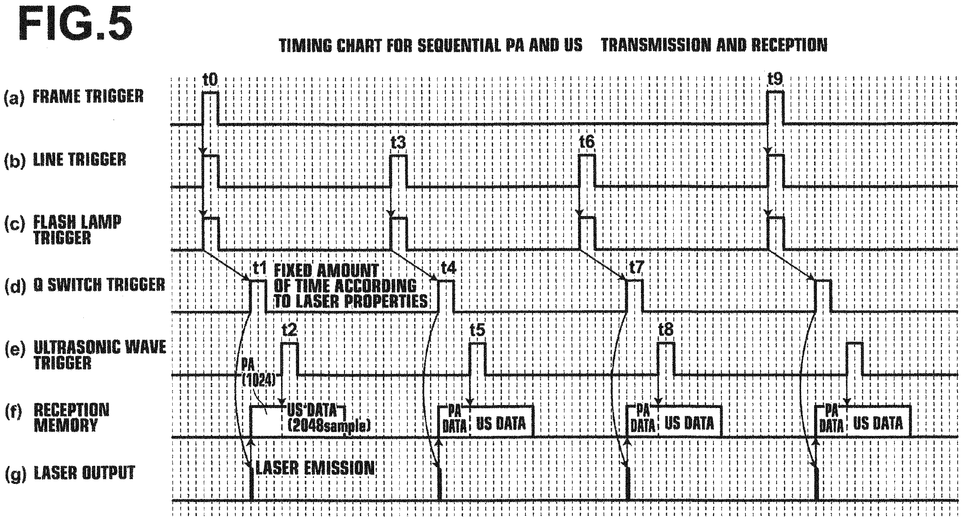

Figure 5 illustrates a timing chart of an exemplary operation. The control means 31 generates a frame trigger signal (a). In addition, the control means 31 generates a line trigger signal (b) having three pulses, corresponding to each of Area A through Area C for a single frame. The areas are selected in order from Area A, Area B, and Area C. The control means 31 outputs a pulse of the frame trigger signal and a first pulse of the line trigger signal at time t0. When the first pulse of the line trigger signal is output, the trigger control circuit 28 outputs a flash lamp trigger signal (c) to the laser unit 13. The laser unit 13 lights the flash lamp 32 and pumps the laser medium.

After outputting the flash lamp trigger signal, the trigger control circuit 28 outputs a Q switch trigger signal (d) at time t1. The amount of time from time t0 to time t1 may be set according to the properties of the laser. The laser unit 13 outputs a pulsed laser beam (g) by the Q switch 33 being turned ON. The receiving circuit 21 receives photoacoustic signals which are detected by the 64 ultrasonic transducers corresponding to Area A. The sampling control circuit 29 outputs a sampling trigger signal to the A/D converting means 22 synchronized with the timing of light irradiation. The A/D converting means 22 receives the sampling trigger signal, initiates sampling of the photoacoustic signals, and stores the photoacoustic signals detected by the 64 ultrasonic transducers corresponding to Area A in the reception memory 23 (f).

The reception memory 23 stores therein sampled data of photoacoustic signals and reflected acoustic signals for a number of data points corresponding to the surface of the subject to a depth position of approximately 40mm. Specifically, the reception memory 23 stores sampled data of photoacoustic signals for 1024 points corresponding to approximately 40mm in the depth direction, and sampled data of reflected acoustic waves for 2048 points corresponding to approximately 40mm in the depth direction. In this case, the trigger control circuit 28 outputs an ultrasonic wave trigger signal (e) at time t2, which is the timing at which the A/D converting means 22 samples a 1024th piece of data from initiation of sampling. When the ultrasonic wave trigger signal is output, the probe 11 transmits ultrasonic waves from the 64 ultrasonic transducers that correspond to Area A, and detects reflected acoustic signals with the 64 ultrasonic transducers that correspond to Area A.

When switching from photoacoustic to ultrasonic waves, the A/D converting means 22 does not interrupt sampling of detected signals, and maintains a sampling state. The A/D converting means 22 stores sampled data of the reflected acoustic signals detected by the 64 ultrasonic transducers that correspond to Area A following the photoacoustic signals in the reception memory 23 (f). The reception memory 23 stores the photoacoustic signals and the reflected acoustic signals for Area A as a series of data. Detection of photoacoustic signals and reflected acoustic signals for Area A is completed when 2048 pieces of sampled data of reflected acoustic signals are stored in the reception memory 23.

When detection within Area A is completed, the control means 31 outputs a second pulse of the line trigger signal (b) at time t3. In response to the output of the second pulse of the line trigger signal, the trigger control circuit 28 outputs a flash lamp trigger signal (c) to the laser unit 13. The laser unit 13 lights the flash lamp 32, and pumps the laser medium. After outputting the flash lamp trigger signal, the trigger control circuit 28 outputs a Q switch trigger signal (d) at time t4. The laser unit 13 outputs a pulsed laser beam (g) by the Q switch 33 being turned ON.

The receiving circuit 21 receives photoacoustic signals which are detected by the 64 ultrasonic transducers corresponding to Area B. The sampling control circuit 29 outputs a sampling trigger signal to the A/D converting means 22 synchronized with the timing of light irradiation. The A/D converting means 22 receives the sampling trigger signal, initiates sampling of the photoacoustic signals, and stores the photoacoustic signals detected by the 64 ultrasonic transducers corresponding to Area B in the reception memory 23 (f). The trigger control circuit 28 outputs an ultrasonic wave trigger signal (e) at time t5, which is the timing at which the A/D converting means 22 samples a 1024th piece of data from initiation of sampling.

The probe 11 transmits ultrasonic waves from the 64 ultrasonic transducers that correspond to Area B, and detects reflected acoustic signals with the 64 ultrasonic transducers that correspond to Area B. The A/D converting means 22 stores sampled data of the reflected acoustic signals detected by the 64 ultrasonic transducers that correspond to Area B following the photoacoustic signals in the reception memory 23 (f). The reception memory 23 stores the photoacoustic signals and the reflected acoustic signals for Area B as a series of data. Detection of photoacoustic signals and reflected acoustic signals for Area B is completed when 2048 pieces of sampled data of reflected acoustic signals are stored in the reception memory 23.

When detection within Area B is completed, the control means 31 outputs a third pulse of the line trigger signal (b) at time t6. In response to the output of the second pulse of the line trigger signal, the trigger control circuit 28 outputs a flash lamp trigger signal (c) to the laser unit 13. The laser unit 13 lights the flash lamp 32, and pumps the laser medium. After outputting the flash lamp trigger signal, the trigger control circuit 28 outputs a Q switch trigger signal (d) at time t7. The laser unit 13 outputs a pulsed laser beam (g) by the Q switch 33 being turned ON.

The receiving circuit 21 receives photoacoustic signals which are detected by the 64 ultrasonic transducers corresponding to Area C. The sampling control circuit 29 outputs a sampling trigger signal to the A/D converting means 22 synchronized with the timing of light irradiation. The A/D converting means 22 receives the sampling trigger signal, initiates sampling of the photoacoustic signals, and stores the photoacoustic signals detected by the 64 ultrasonic transducers corresponding to Area B in the reception memory 23 (f). The trigger control circuit 28 outputs an ultrasonic wave trigger signal (e) at time t8, which is the timing at which the A/D converting means 22 samples a 1024th piece of data from initiation of sampling.

The probe 11 transmits ultrasonic waves from the 64 ultrasonic transducers that correspond to Area C, and detects reflected acoustic signals with the 64 ultrasonic transducers that correspond to Area C. The A/D converting means 22 stores sampled data of the reflected acoustic signals detected by the 64 ultrasonic transducers that correspond to Area C following the photoacoustic signals in the reception memory 23 (f). The reception memory 23 stores the photoacoustic signals and the reflected acoustic signals for Area C as a series of data. Detection of photoacoustic signals and reflected acoustic signals for Area C is completed when 2048 pieces of sampled data of reflected acoustic signals are stored in the reception memory 23.

Data necessary to generate a photoacoustic image and an ultrasound image are collected in the reception memory 23 by detecting the photoacoustic signals and the reflected acoustic signals in the three areas A through C. The data separating means 24 separates the photoacoustic signals and the reflected acoustic signals in the reception memory 23. The data separating means 24 reads out 1024 pieces of sampled data within a specific address range from among the 3072 pieces of sampled data for each element as photoacoustic signals, and reads out the remaining 2048 pieces of sampled data as reflected acoustic signals to separate the photoacoustic signals and the reflected acoustic signals, for example. Alternatively, a delimiter may be recorded between the photoacoustic signals and the reflected acoustic signals as a data breakpoint, and the photoacoustic signals and the reflected acoustic signals may be separated employing the delimiter. As a further alternative, header data may be added to the photoacoustic signals and the reflected acoustic signals within the reception memory 23, and the signals may be separated by referring to the header data. The photoacoustic image generating means 25 generates a photoacoustic image based on photoacoustic signals having 1024 data points for each element. Meanwhile, the ultrasound image generating means 26 employs the 1/2 resampling means 261 to resample the reflected acoustic signals having 2048 data points for each element into 1024 data points, and then generates an ultrasound image.

When detection (sampling) of the photoacoustic signals and the reflected acoustic signals for Area C is completed, the control means 31 outputs a pulse of a frame trigger signal (a) at time t9. The operations following thereafter are the same as those following output of the first pulse of the frame trigger signal at time t0. The tomographic image generating apparatus 10 generates photoacoustic images and ultrasound images, by irradiating light, detecting photoacoustic signals, transmitting ultrasonic waves, and detecting reflected acoustic signals within each area for each frame.

Note that in the case that division into regions is not performed, photoacoustic signals from the light absorber 51 may be detected by all 192 elements after the laser beam is irradiated onto the subject. In addition, ultrasonic waves may be transmitted from all 192 elements, and reflected acoustic signals from the reflector 52 may be detected by all 192 elements. The division into areas is not limited to that described above, and the number of divided areas may be less than three or greater than three. Although there is no overlap among the areas in the case described above, the areas may overlap each other.

In the present embodiment, light is irradiated onto a subject, sampling of ultrasonic wave signals (photoacoustic signals) from the subject is initiated, and the sampled photoacoustic signals are stored in the reception memory 23. In addition, ultrasonic wave transmission is executed while maintaining the ultrasonic wave signal sampling state, ultrasonic wave signals (reflected acoustic waves) from the subject are sampled, and stored in the reception memory 23. In the present embodiment, the reception memory 23 is employed as a common memory for the photoacoustic signals and the reflected acoustic signals. The A/D converting means 22 samples the reflected acoustic signals after sampling the photoacoustic signals, without interrupting the sampling operation. The photoacoustic signals and reflected acoustic signals can be obtained seamlessly by adopting configuration, and the amount of time required from initiation of obtainment of the photoacoustic signals to completion of obtainment of the reflected acoustic signals can be shortened.

A case in which photoacoustic signals are stored in a memory for photoacoustic signals and reflected acoustic signals are stored in a memory for reflected acoustic signals will be considered as a comparative example. In this case, it is necessary for the A/D converting means 22 to switch the memory in which sampled data is to be stored when transitioning from sampling of photoacoustic signals to sampling of reflected acoustic signals. In order to switch memories, it is necessary to interrupt a sampling operation at a point in time at which obtainment of photoacoustic signals is completed. This results in the amount of time required from initiation of obtainment of the photoacoustic signals to completion of obtainment of the reflected acoustic signals to become wastefully long. The present embodiment employs a common memory for both types of signals, and transmits ultrasonic waves while maintaining a sampling state. Therefore, the amount of time required to obtain data can be shortened compared to the comparative example, and processing can be accelerated. By accelerating processing speed, smooth video can be displayed at an improved frame rate when repeatedly generating photoacoustic images and ultrasound images and displaying them as a video, for example.

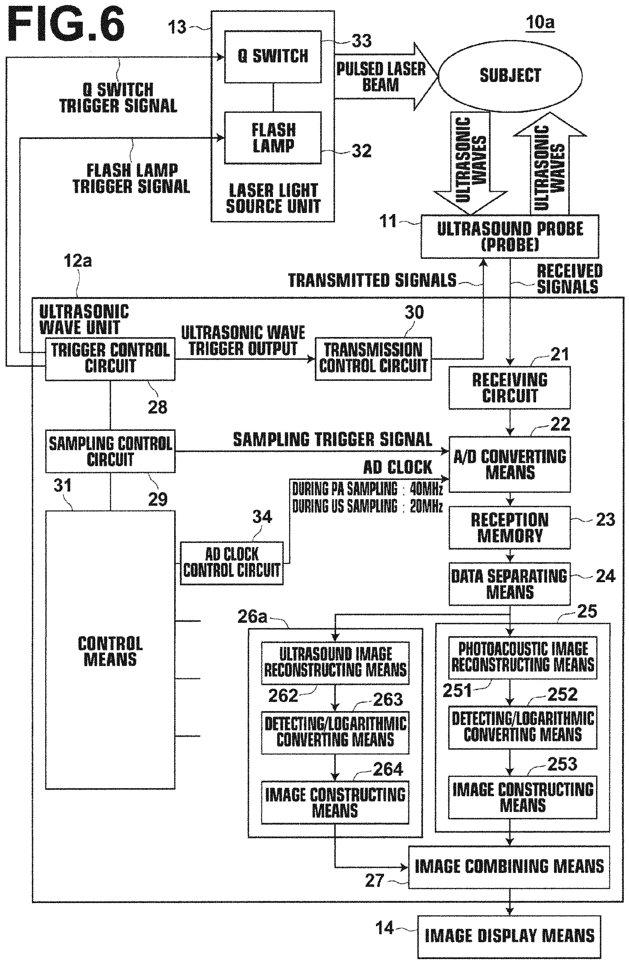

Next, a second embodiment of the present invention will be described. Figure 6 illustrates a tomographic image generating apparatus 10 according to the second embodiment of the present invention. The configuration of an ultrasonic wave unit 12a of the tomographic image generating apparatus 10 of the present embodiment differs from the tomographic image generating apparatus 10 of the first embodiment illustrated in Figure 1. The ultrasonic wave unit 12a further comprises an A/D clock control circuit (sampling rate control means) 34. In addition, the configuration of an ultrasound image generating means 26a of the present embodiment is that of the ultrasound image generating means 26 of the first embodiment, from which the 1/2 sampling means 261 is omitted.

The A/D clock control circuit 34 controls the sampling rate of the A/D converting means 22 by controlling the frequency of the A/D clock signal input to the A/D converting means 22, for example. The A/D clock control circuit 34 controls the sampling rate when the A/D converting means samples reflected acoustic signals to be half the sampling rate when sampling photoacoustic signals. For example, the A/D clock control circuit 34 outputs an A/D clock signal having a clock frequency of 40MHz when the A/D converting means 22 is sampling photoacoustic signals, to cause the A/D converting means 22 to sample the photoacoustic signals at a sampling rate of 40M samples/second. When the A/D converting means 22 is sampling reflected acoustic signals, the A/D clock control circuit 34 outputs an A/D clock signal having a clock frequency of 20MHz, to cause the A/D converting means 22 to sample the reflected acoustic signals at a sampling rate of 20M samples/second. The A/D clock control circuit 34 decreases the frequency of the A/D clock signal to half synchronized with a timing at which the trigger control circuit 28 outputs an ultrasonic wave trigger signal, for example.

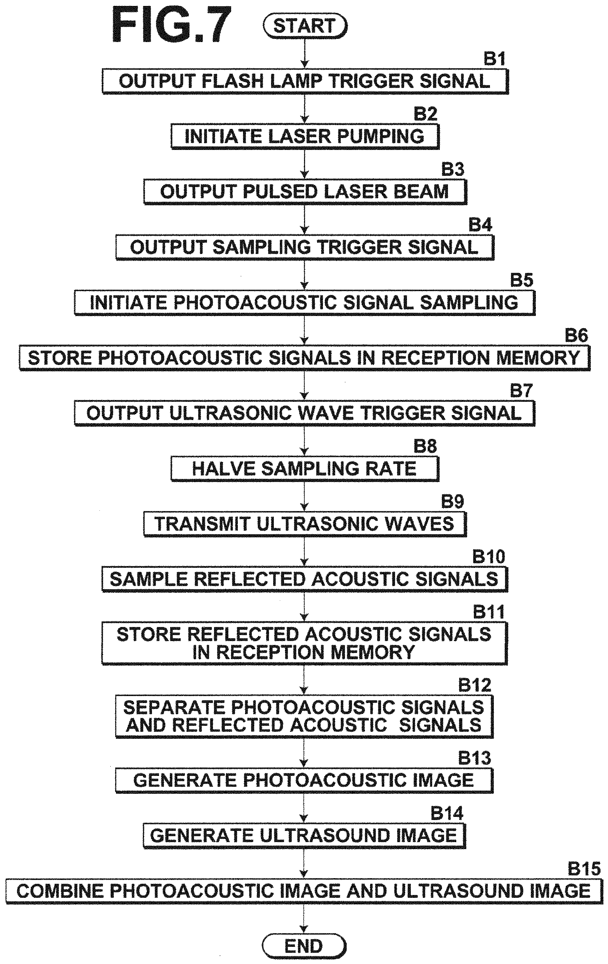

Figure 7 illustrates operational procedures of the tomographic image generating apparatus 10a of the second embodiment. The trigger control circuit 28 outputs a flash lamp trigger signal to the laser unit 13 (step B1). The flash lamp 32 of the laser unit 13 is lit in response to the flash lamp trigger signal, and pumping of the laser medium is initiated (step B2). The trigger control circuit 28 outputs a Q switch trigger signal to the laser unit 13 to turn the Q switch ON, thereby causing a pulsed laser bema to be output from the laser unit 13 (step B3). The laser beam output from the laser unit 13 is irradiated onto a subject. Photoacoustic signals are generated within the subject due to the irradiated pulsed laser beam. The probe 11 detects the photoacoustic signals generated within the subject.

The sampling control circuit 29 sends a sampling trigger signal to the A/D converting means 22 synchronized with the timing at which the laser is output (step B4). The photoacoustic signals detected by the probe 11 are input to the A/D converting means 22 via the receiving circuit 21, and the A/D converting means 22 initiates sampling of the photoacoustic signals (step B5). At this time, an A/D clock signal having a clock frequency of 40MHz, for example, is being input to the A/D converting means 22 by the A/D clock control circuit 34. The A/D converting means 22 samples the photoacoustic signals at a sampling rate of 40M (samples)/second, based on the A/D clock signal. The A/D converting means 22 stores the sampled photoacoustic signals in the reception memory 23 (step B6).

The trigger control circuit 28 outputs an ultrasonic wave trigger signal at a predetermined timing (step B7). The trigger control circuit 28 outputs the ultrasonic wave trigger signal at a timing at which the number of pieces of sampled data sampled from initiation of sampling by the A/D converting means 22 reaches a predetermined number. In other words, the trigger control circuit 28 outputs the ultrasonic wave trigger signal at a timing after a predetermined amount of time has elapsed form the timing that the sampling control circuit 29 outputs the sampling trigger signal. At this time, the A/D converting means 22 does not interrupt sampling, and maintains a sampling state. The steps up to this point may be the same as the operational procedures of the first embodiment illustrated in Figure 2.

The A/D clock control circuit 34 controls the sampling rate of the A/D converting means 22 to half the sampling rate when sampling the photoacoustic signals, synchronized with the timing at which ultrasonic waves are transmitted (step B8). In other words, the sampling rate of the A/D converting means 22 is decreased to half synchronized with a timing at which detection of reflected acoustic signals is initiated. The A/D clock control circuit 34 changes the clock frequency of the A/D clock signal input to the A/D converting means 22 from 40MHz to 20MHz, for example, to decrease the sampling rate of the A/D converting means 22 by half.

When the ultrasonic wave trigger signal that commands transmission of ultrasonic waves is received via the transmission control circuit 30, the probe 11 transmits ultrasonic waves toward the subject (step B9). The probe 11 detects reflected acoustic signals of the transmitted ultrasonic waves after transmitting the ultrasonic waves. The A/D converting means 22 samples the reflected acoustic signals continuous with sampling of the photoacoustic signals (step B10). At this time, the A/D clock control circuit 34 is controlling the sampling rate of the A/D converting means 22 to half. Therefore, the A/D converting means 22 samples the reflected acoustic signals at a sampling rate which is half the sampling rate when sampling the photoacoustic signals. The A/D converting means 22 stores the sampled reflected acoustic signals in the reception memory 23 continuous with the stored photoacoustic signals. Sampling is completed when the A/D converting means 22 samples a predetermined number of reflected acoustic signals.

The data separating means 24 separates the photoacoustic signals and the ultrasonic wave signals from the reception memory 23, in which the photoacoustic signals and the ultrasonic wave signals are continuously stored, provides the photoacoustic signals to the photoacoustic image generating means 25, and provides the ultrasonic wave signals to the ultrasound image generating means 26 (step B12). The photoacoustic image generating means 25 generates a photoacoustic image based on the photoacoustic signals (step B13). Separation of the data and generation of the photoacoustic image may be performed in the same manner as in the first embodiment. The ultrasound image generating means 26 generates an ultrasound image based on the reflected acoustic signals (step B14). In the present embodiment, the sampling rate for the reflected acoustic signals is half the sampling rate for the photoacoustic signals. Therefore, it is not necessary to resample the data points to half by employing the 1/2 resampling means 261 (Figure 1) when generating the ultrasound image. The image combining means 27 combines the photoacoustic image and the ultrasound image (step B15), and displays the combined image on the display screen of the image display means 14.

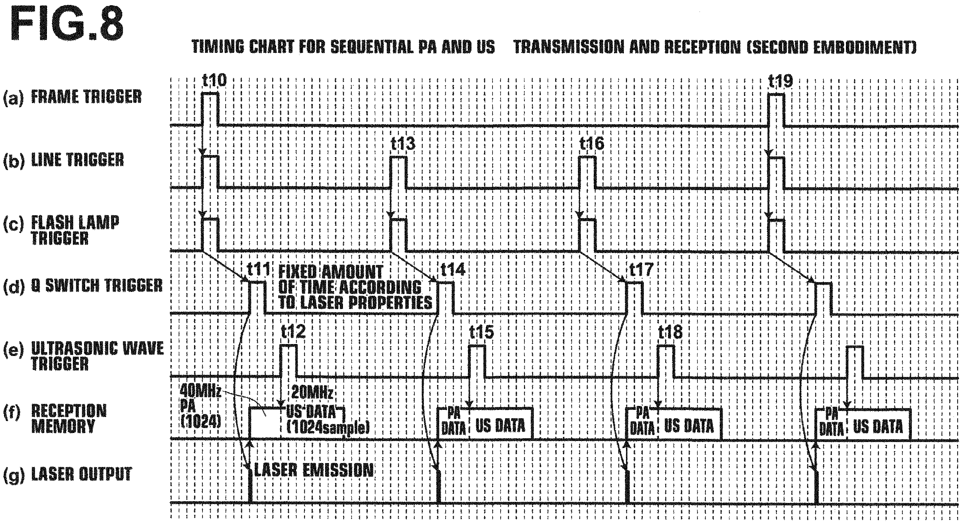

Figure 8 illustrates a timing chart of an exemplary operation. Here, the range in which the ultrasonic transducers of the probe 11 are arranged is divided into three regions, Area A, Area B, and Area C as illustrated in Figure 3, and photoacoustic signals and reflected acoustic signals are detected in order from Area A, Area B, and then Area C. The control means 31 generates a frame trigger signal (a). In addition, the control means 31 generates a line trigger signal (b) having three pulses, corresponding to each of Area A through Area C for a single frame. The control means 31 outputs a pulse of the frame trigger signal and a first pulse of the line trigger signal at time t10. When the first pulse of the line trigger signal is output, the trigger control circuit 28 outputs a flash lamp trigger signal (c) to the laser unit 13. The laser unit 13 lights the flash lamp 32 and pumps the laser medium.

After outputting the flash lamp trigger signal, the trigger control circuit 28 outputs a Q switch trigger signal (d) at time t11. The amount of time from time t10 to time t11 may be set according to the properties of the laser. The laser unit 13 outputs a pulsed laser beam (g) by the Q switch 33 being turned ON. The receiving circuit 21 receives photoacoustic signals which are detected by the 64 ultrasonic transducers corresponding to Area A. The sampling control circuit 29 outputs a sampling trigger signal to the A/D converting means 22 synchronized with the timing of light irradiation. The A/D converting means 22 receives the sampling trigger signal, initiates sampling of the photoacoustic signals, and stores the photoacoustic signals detected by the 64 ultrasonic transducers corresponding to Area A in the reception memory 23 (f).

The reception memory 23 stores therein sampled data of photoacoustic signals and reflected acoustic signals for a number of data points corresponding to the surface of the subject to a depth position of approximately 40mm. Specifically, the reception memory 23 stores sampled data of photoacoustic signals for 1024 points corresponding to approximately 40mm in the depth direction. In this case, the trigger control circuit 28 outputs an ultrasonic wave trigger signal (e) at time t12, which is the timing at which the A/D converting means 22 samples a 1024th piece of data from initiation of sampling. When the ultrasonic wave trigger signal is output, the probe 11 transmits ultrasonic waves from the 64 ultrasonic transducers that correspond to Area A, and detects reflected acoustic signals with the 64 ultrasonic transducers that correspond to Area A. At this time, the A/D clock control circuit 34 changes the clock frequency of the A/D clock signal which is output to the A/D converting means 22 from 40MHz to 20MHz.

When switching from photoacoustic to ultrasonic waves, the A/D converting means 22 does not interrupt sampling of detected signals, and maintains a sampling state. However, because the clock frequency of the A/D clock signal is half that when sampling the photoacoustic signals, the sampling rate becomes half that when sampling the photoacoustic signals. The A/D converting means 22 stores sampled data of the reflected acoustic signals detected by the 64 ultrasonic transducers that correspond to Area A following the photoacoustic signals in the reception memory 23 (f).

The sampling rate for the reflected acoustic signals is half the sampling rate for the photoacoustic signals. Therefore, in the case that the same number of pieces of sampled data as the photoacoustic signals is to be obtained, sampling of the reflected acoustic signals will take twice the amount of time as that required to sample the photoacoustic signals. In the case that photoacoustic signals and reflected acoustic signals are sampled at the same sampling rate, the number of data points of reflected acoustic signals corresponding to approximately 40mm in the depth direction will become 2048 (refer to Figure 5). In the present embodiment, the sampling rate is reduced to half. Therefore, the number of data points of reflected acoustic signals corresponding to approximately 40mm in the depth direction is 1024. Detection of photoacoustic signals and reflected acoustic signals for Area A is completed when 1024 pieces of sampled data of reflected acoustic signals are stored in the reception memory 23.

When detection within Area A is completed, the control means 31 outputs a second pulse of the line trigger signal (b) at time t13. In response to the output of the second pulse of the line trigger signal, the trigger control circuit 28 outputs a flash lamp trigger signal (c) to the laser unit 13. The laser unit 13 lights the flash lamp 32, and pumps the laser medium. After outputting the flash lamp trigger signal, the trigger control circuit 28 outputs a Q switch trigger signal (d) at time t14. The laser unit 13 outputs a pulsed laser beam (g) by the Q switch 33 being turned ON.

The receiving circuit 21 receives photoacoustic signals which are detected by the 64 ultrasonic transducers corresponding to Area B. The sampling control circuit 29 outputs a sampling trigger signal to the A/D converting means 22 synchronized with the timing of light irradiation. At this time, the A/D clock control circuit 34 is outputting an A/D clock signal having a clock frequency of 40MHz to the A/D converting means 22. The A/D converting means 22 receives the sampling trigger signal, initiates sampling of the photoacoustic signals at a sampling rate of 40M samples/second, and stores the photoacoustic signals detected by the 64 ultrasonic transducers corresponding to Area B in the reception memory 23 (f). The trigger control circuit 28 outputs an ultrasonic wave trigger signal (e) at time t15, which is the timing at which the A/D converting means 22 samples a 1024th piece of data from initiation of sampling. At this time, the A/D clock control circuit 34 changes the clock frequency of the A/D clock signal which is output to the A/D converting means 22 from 40MHz to 20MHz.

The probe 11 transmits ultrasonic waves from the 64 ultrasonic transducers that correspond to Area B, and detects reflected acoustic signals with the 64 ultrasonic transducers that correspond to Area B. The A/D converting means 22 samples the reflected acoustic signals detected by the 64 ultrasonic transducers that correspond to Area B at a sampling rate of 20M samples/second, and stores the sampled data in the reception memory 23 (f) following the photoacoustic signals. The reception memory 23 stores the photoacoustic signals and the reflected acoustic signals for Area B as a series of data. Detection of photoacoustic signals and reflected acoustic signals for Area B is completed when 1024 pieces of sampled data of reflected acoustic signals are stored in the reception memory 23.

When detection within Area B is completed, the control means 31 outputs a third pulse of the line trigger signal (b) at time t16. In response to the output of the second pulse of the line trigger signal, the trigger control circuit 28 outputs a flash lamp trigger signal (c) to the laser unit 13. The laser unit 13 lights the flash lamp 32, and pumps the laser medium. After outputting the flash lamp trigger signal, the trigger control circuit 28 outputs a Q switch trigger signal (d) at time t17. The laser unit 13 outputs a pulsed laser beam (g) by the Q switch 33 being turned ON.

The receiving circuit 21 receives photoacoustic signals which are detected by the 64 ultrasonic transducers corresponding to Area C. The sampling control circuit 29 outputs a sampling trigger signal to the A/D converting means 22 synchronized with the timing of light irradiation. At this time, the A/D clock control circuit 34 is outputting an A/D clock signal having a clock frequency of 40MHz to the A/D converting means 22. The A/D converting means 22 receives the sampling trigger signal, initiates sampling of the photoacoustic signals at a sampling rate of 40M samples/second, and stores the photoacoustic signals detected by the 64 ultrasonic transducers corresponding to Area C in the reception memory 23 (f). The trigger control circuit 28 outputs an ultrasonic wave trigger signal (e) at time t18, which is the timing at which the A/D converting means 22 samples a 1024th piece of data from initiation of sampling. At this time, the A/D clock control circuit 34 changes the clock frequency of the A/D clock signal which is output to the A/D converting means 22 from 40MHz to 20MHz.

The probe 11 transmits ultrasonic waves from the 64 ultrasonic transducers that correspond to Area C, and detects reflected acoustic signals with the 64 ultrasonic transducers that correspond to Area B. The A/D converting means 22 samples the reflected acoustic signals detected by the 64 ultrasonic transducers that correspond to Area C at a sampling rate of 20M samples/second, and stores the sampled data in the reception memory 23 (f) following the photoacoustic signals. The reception memory 23 stores the photoacoustic signals and the reflected acoustic signals for Area B as a series of data. Detection of photoacoustic signals and reflected acoustic signals for Area C is completed when 1024 pieces of sampled data of reflected acoustic signals are stored in the reception memory 23.

Data necessary to generate a photoacoustic image and an ultrasound image are collected in the reception memory 23 by detecting the photoacoustic signals and the reflected acoustic signals in the three areas A through C. The data separating means 24 separates the photoacoustic signals and the reflected acoustic signals in the reception memory 23. The data separating means 24 reads out 1024 pieces of sampled data within a specific address range from among the 2048 pieces of sampled data for each element as photoacoustic signals, and reads out the remaining 1024 pieces of sampled data as reflected acoustic signals to separate the photoacoustic signals and the reflected acoustic signals, for example. The photoacoustic image generating means 25 generates a photoacoustic image based on photoacoustic signals having 1024 data points for each element. Meanwhile, the ultrasound image generating means 26 generates an ultrasound image based on reflected acoustic signals having 1024 data points for each element.

When detection (sampling) of the photoacoustic signals and the reflected acoustic signals for Area C is completed, the control means 31 outputs a pulse of a frame trigger signal (a) at time t19. The operations following thereafter are the same as those following output of the first pulse of the frame trigger signal at time t10. The tomographic image generating apparatus 10 generates photoacoustic images and ultrasound images, by irradiating light, detecting photoacoustic signals, transmitting ultrasonic waves, and detecting reflected acoustic signals within each area for each frame.

In the present embodiment, the A/D clock control circuit 34 controls the sampling rate when the A/D converting means 22 detects the reflected acoustic signals to be half the sampling rate when detecting the photoacoustic signals. Thereby, photoacoustic images and ultrasound images can be imaged up to the same position in the depth direction, even if the number of data points to be stored in the reception memory 23 is the same for the photoacoustic signals and the reflected acoustic signals. The present embodiment can reduce the number of sampled data points of reflected acoustic signals to be stored in the reception memory 23 to half that of the first embodiment. As a result, the necessary capacity of the reception memory 23 can be reduced. The other advantageous effects are the same as those obtained by the first embodiment.

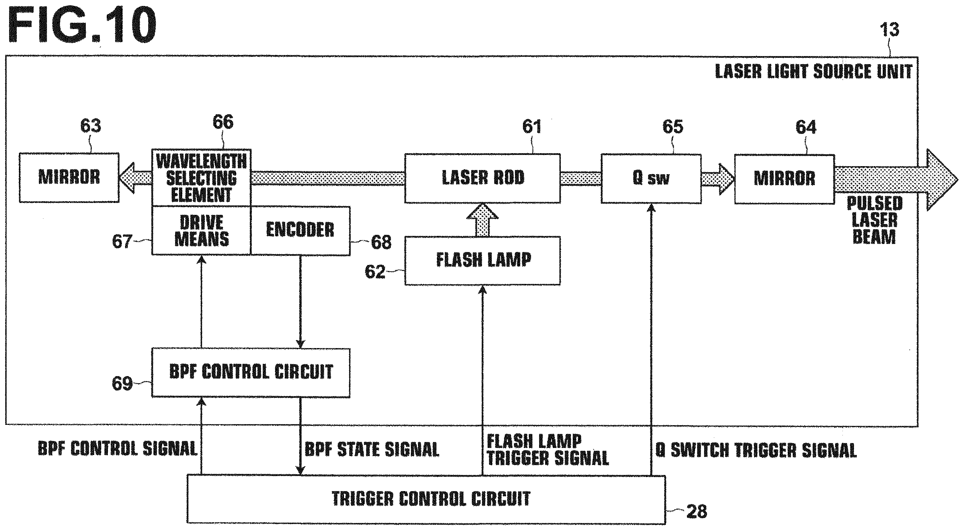

Next, a third embodiment of the present invention will be described. Figure 10 illustrates a tomographic image generating apparatus 10b according to the third embodiment of the present invention. The tomographic image generating apparatus 10b is equipped with an ultrasound probe (probe) 11, an ultrasonic wave unit 12b, and a light source (laser unit) 13. The ultrasonic wave unit 12b has a receiving circuit 21, an A/D converting means 22, a reception memory 23, a data separating means 24, a photoacoustic image generating means 25b, an ultrasound image generating means 26, an image combining means 27, a trigger control circuit 28, a sampling control circuit 29, a transmission control circuit 30, and a control means 31. In the present embodiment, the laser unit 13 irradiates a plurality of laser beams having different wavelengths from each other onto a subject. The photoacoustic image generating means 25b utilizes wavelength dependent properties of light absorption characteristics for light absorbers within subjects to generate photoacoustic images in which arteries and veins can be distinguished, for example.

The laser unit 13 of the present embodiment switches output of a plurality of pulsed laser beams having different wavelengths from each other. The pulsed laser beams output from the laser unit 13 are guided to the probe 11 using a light guiding means such as an optical fiber, then irradiated onto a subject from the probe 11. The following description will mainly be of a case in which the laser unit is capable of outputting a pulsed laser beam having a first wavelength and a pulsed laser beam having a second wavelength.

A case will be considered in which the first wavelength (central wavelength) is approximately 750nm, and the second wavelength is approximately 800nm. The molecular absorption coefficient of oxidized hemoglobin (hemoglobin bound to oxygen: oxy-Hb), which is contained in human arteries, for a wavelength of 750nm is greater than that for a wavelength of 800nm. Meanwhile, molecular absorption coefficient of deoxidized hemoglobin (hemoglobin not bound to oxygen: deoxy-Hb), which is contained in veins, for a wavelength of 750nm is less than that for a wavelength of 800nm. Photoacoustic signals from arteries and photoacoustic signals from veins can be distinguished by checking the relative intensities of photoacoustic signals obtained for a wavelength of 800nm and photoacoustic signals obtained for a wavelength of 750nm, utilizing these characteristics.

The probe 11 detects acoustic waves (photoacoustic waves or reflected acoustic waves) from within subjects. The receiving circuit 21 receives detected signals of acoustic waves received by the probe 11. The A/D converting means 22 samples the detected signals received by the receiving circuit 21. The A/D converting means 22 samples the ultrasonic signals at a predetermined sampling period synchronized with an A/D clock signal, for example. The A/D converting means 22 stores reflected acoustic data obtained by sampling reflected acoustic signals and photoacoustic data obtained by sampling photoacoustic signals in the reception memory 23.

The trigger control circuit 28 outputs a light trigger signal that commands light output to the laser unit 13. The trigger control circuit 28 first outputs a flash lamp trigger signal, then outputs a Q switch trigger signal thereafter. The laser unit 13 pumps a laser medium in response to the flash lamp trigger signal, and outputs a pulsed laser beam in response to the Q switch trigger signal. The timing of the Q switch trigger may be generated within the laser unit 13 instead of the Q switch trigger signal being sent to the laser unit 13 from the trigger control circuit 28. In this case, a signal that indicates that a Q switch has been turned ON may be transmitted to the ultrasonic wave unit 12b from the laser unit 13. Here, the light trigger signal is a concept that includes at least one of the flash lamp trigger signal and the Q switch trigger signal. The Q switch trigger signal corresponds to the light trigger signal in the case that the trigger control circuit 28 outputs the Q switch trigger signal. The flash lamp trigger signal corresponds to the light trigger signal in the case that the laser unit 13 generates the timing of the Q switch trigger.

In addition, the trigger control circuit 28 outputs an ultrasonic wave trigger signal that commands ultrasonic wave transmission to the transmission control circuit 30. When the trigger signal is received, the transmission control circuit 30 causes the probe 11 to transmit ultrasonic waves. The trigger control circuit 28 outputs a light trigger signal first, and then outputs an ultrasonic wave trigger signal, for example. Irradiation of a laser beam and detection of photoacoustic signals are performed by the light trigger signal being output, and transmission of ultrasonic waves toward a subject and detection of reflected acoustic signals are performed thereafter by output of the ultrasonic wave trigger signal.

The sampling control circuit 29 outputs a sampling trigger signal that commands initiation of sampling to the A/D converting means 22. the sampling control circuit 29 outputs a sampling trigger signal at a timing following output of a light trigger signal by the trigger control circuit 28 and prior to output of an ultrasonic wave trigger signal. The sampling control circuit 29 outputs a sampling trigger signal at a timing following output of the light trigger signal, and preferably at a timing at which a laser beam is actually irradiated onto a subject. For example, the sampling control circuit 29 outputs a sampling trigger signal synchronized with the timing at which the trigger control circuit 28 outputs a Q switch trigger signal. When the sampling trigger signal is received, the A/D converting means 22 initiates sampling of ultrasonic waves (photoacoustic signals) detected by the probe 11.

Following output of the light trigger signal, the trigger control circuit 28 outputs an ultrasonic wave trigger signal at a timing that detection of photoacoustic signals is completed. At this time, the A/D converting means 22 does not interrupt sampling of ultrasonic wave signals, but continues to execute sampling. In other words, the trigger control circuit 28 outputs the ultrasonic wave trigger signal in a state in which the A/D converting means 22 is continuing sampling of the ultrasonic wave signals. The ultrasonic waves detected by the probe 11 change from photoacoustic waves to reflected acoustic waves, by the probe 11 transmitting ultrasonic waves in response to the ultrasonic wave trigger signal. The A/D converting means 22 continuously samples the photoacoustic waves and the reflected acoustic waves, by continuing sampling of detected ultrasonic wave signals.

The A/D converting means 22 stores both the sampled photoacoustic signals and the sampled reflected acoustic signals in the common reception memory 23. A semiconductor memory device, for example, may be employed as the reception memory. Other memory devices, such as a magnetic memory device, may also be employed as the reception memory 23. The sampled data stored in the reception memory 23 are data of photoacoustic signals up to a certain point in time, and become data of reflected acoustic signals after the point in time.

Sampling of photoacoustic signals is repeated for the number of wavelengths of light output by the laser unit 13. For example, first, the light beam having the first wavelength is irradiated onto a subject from the laser unit 13, and first photoacoustic signals (first photoacoustic data) detected by the probe 11 when the pulsed laser beam having the first wavelength is irradiated onto the subject are stored in the reception memory 23. Next, the light beam having the second wavelength is irradiated onto the subject from the laser unit 13, and second photoacoustic signals (second photoacoustic data) detected by the probe 11 when the pulsed laser beam having the second wavelength is irradiated onto the subject are stored in the reception memory 23. Reflected acoustic data are then stored in the reception memory 23 continuous with the second photoacoustic data.

The data separating means 24 separates the ultrasonic wave data, the first photoacoustic data, and the second photoacoustic data, which are stored in the reception memory 23. The data separating means 24 provides the first and second photoacoustic data to the photoacoustic image generating means 25b. The data separating means 24 provides the ultrasonic wave data to the ultrasound image generating means 26. Ultrasound images may be generated by the ultrasound image generating means 26 in the same manner as in the first embodiment.

The photoacoustic image generating means 25b has a photoacoustic image reconstructing means 251, a detecting/logarithmic converting means 252, a photoacoustic image constructing means 253, a two wavelength data complexifying means 254, an intensity data extracting means 255, and a two wavelength data calculating means 256. The two wavelength data complexifying means 254 generates complex number data, in which one of the first photoacoustic signals and the second photoacoustic signals is designated as a real part, and the other is designated as an imaginary part. Hereinafter, a case will be described in which the two wavelength data complexifying means 254 designates the first photoacoustic signals as the real part and the second photoacoustic signals as the imaginary part.

The complex number data, which are the photoacoustic data, are input to the photoacoustic image reconstructing means 251 from the two wavelength data complexifying means 254. The photoacoustic image reconstructing means 251 reconstructs the photoacoustic data. The photoacoustic image reconstructing means 251 reconstructs images from the input complex number data by the Fourier transform method (FTA method). Known techniques, such as that disclosed in J. I. Sperl, et al., "Photoacoustic Image Reconstruction - A Quantitative Analysis", SPIE-OSA, Vol. 6631, 663103, 2007, may be applied to image reconstruction by the Fourier transform method. The photoacoustic image reconstructing means 251 inputs data, which have undergone Fourier transform and represent reconstructed images, to the intensity data extracting means 255 and the two wavelength data calculating means 256.

The two wavelength data calculating means 256 extracts the relative signal intensities between the photoacoustic data corresponding to each wavelength. In the present embodiment, the reconstructed images reconstructed by the photoacoustic image reconstructing means 251 are input to the two wavelength data calculating means 256. The two wavelength data calculating means 256 extracts phase data that represent which of the real part and the imaginary part is larger and by how much, by comparing the real part and the imaginary part of the input data, which are complex number data. When the complex number data is represented by X+iY, for example, the two wavelength data calculating means 256 generates θ=tan⁻¹ (Y/X) as the phase data. Note that θ=90° in the case that X-0. When the first photoacoustic data (X) that constitutes the real part and the second photoacoustic data (Y) that constitutes the imaginary part are equal, the phase data is θ=45°. The phase data becomes closer to θ=0° as the first photoacoustic data is relatively larger, and becomes closer to θ=90° as the second photoacoustic data is relatively larger.

The intensity data extracting means 255 generates intensity data that represent signal intensities, based on the photoacoustic data corresponding to each wavelength. In the present embodiment, the reconstructed images reconstructed by the photoacoustic image reconstructing means 251 are input to the intensity data extracting means 255. The intensity data extracting means 255 generates the intensity data from the input data, which are complex number data. When the complex number data is represented by X+iY, for example, the intensity data extracting means 255 extracts (X²+Y²)^{1/2} as the intensity data.

The phase data from the two wavelength data calculating means 256 and the intensity data, which have undergone the detection/logarithmic conversion process administered by the detecting/logarithmic converting means 252, are input to the photoacoustic image constructing means 253. The photoacoustic image constructing means 253 generates a photoacoustic image, which is a distribution image of light absorbers, based on the input phase data and intensity data. The photoacoustic image constructing means 253 determines the brightness (gradation value) of each pixel within the distribution image of light absorbers, based on the input intensity data, for example. In addition, the photoacoustic image constructing means 253 determines the color (display color) of each pixel within the distribution image of light absorbers, based on the phase data, for example. The photoacoustic image constructing means 253 employs a color map, in which predetermined colors correspond to a phase range from 0° to 90°, to determine the color of each pixel based on the input phase data for example for example.

Here, the phase range from 0° to 45° is a range in which the first photoacoustic data is greater than the second photoacoustic data. Therefore, the source of the photoacoustic signals may be considered to be arteries, through which blood that mainly contains oxidized hemoglobin having greater absorption with respect to a wavelength of 756nm than a wavelength of 798nm flows. Meanwhile, the phase range from 45° to 90° is a range in which the second photoacoustic data is greater than the first photoacoustic data. Therefore, the source of the photoacoustic signals may be considered to be veins, through which blood that mainly contains deoxidized hemoglobin having lower absorption with respect to a wavelength of 798nm than a wavelength of 756nm flows.

Therefore, a color map, in which a phase of 0° corresponds to red that gradually becomes colorless (white) as the phase approaches 45°, and a phase of 90° corresponds to blue that gradually becomes white as the phase approaches 45°, is employed. In this case, portions corresponding to arteries within the photoacoustic image can be displayed red, and portions corresponding to veins can be displayed blue. A configuration may be adopted, wherein the intensity data are not employed, the gradation values are set to be constant, and portions corresponding to arteries and portions corresponding to veins are merely separated by colors according to the phase data.

The image combining means 27 combines the photoacoustic image generated by the photoacoustic image constructing means 253 and the ultrasound image generated by the ultrasound image generating means 26. The combined image is displayed by the image display means. Alternatively, it is possible for the image display means 14 to display the photoacoustic image and the ultrasound image arranged next to each other or to switch display between the photoacoustic image and the ultrasound image, without combining the images.

Next, the configuration of the laser unit 13 will be described in detail. Figure 10 illustrates the construction of the laser unit 13. The laser unit 13 has: a laser rod 61, a flash lamp 62, mirrors 63 and 64, a Q switch 65, a wavelength selecting element 66, a drive means 67, a driving state detecting means 68, and a BPF control circuit 69. The flash lamp 62 and the Q switch 65 correspond to the flash lamp 32 and the Q switch 33 of Figure 1, respectively.

The laser rod 61 is a laser medium. An alexandrite crystal, a Cr:LiSAF (Cr:LiSrAlF6), Cr:LiCAF (Cr:LiCaAlF6) crystal, or a Ti: Sapphire crystal may be employed as the laser rod 61. The flash lamp 62 is a pumping light source, and irradiates pumping light onto the laser rod 61. Light sources other than the flash lamp 62, such as semiconductor lasers and solid state lasers, may be employed as the pumping light source.

The mirrors 63 and 64 face each other with the laser rod 61 sandwiched therebetween. The mirrors 63 and 64 constitute an optical resonator. Here, the mirror 64 is an output side mirror. The Q switch 65 is inserted within the resonator. The Q switch 65 changes the insertion loss within the optical resonator from high loss (low Q) to low loss (high Q) at high speed, to obtain a pulsed laser beam.

The wavelength selecting element 66 includes a plurality of band pass filters (BPF: Band Pass Filters) that transmit wavelengths different from each other. The wavelength selecting element 66 selectively inserts the plurality of band pass filters into the optical path of the optical resonator. The wavelength selecting element 66 includes a first band pass filter that transmits light having a wavelength of 750nm (central wavelength) and a second band pass filter that transmits light having a wavelength of 800nm (central wavelength), for example. The oscillating wavelength of the laser beam oscillator can be set to 750nm by inserting the first band pass filter into the optical path of the optical oscillator, and the oscillating wavelength of the laser beam oscillator can be set to 800nm by inserting the second band pass filter into the optical path of the optical oscillator.

The drive means 67 drives the wavelength selecting element 66 such that the band pass filters which are inserted into the optical path of the optical resonator are sequentially switched in a predetermined order. For example, if the wavelength selecting element 66 is constituted by a rotatable filter body that switches the band pass filter to be inserted into the optical path of the optical resonator by rotational displacement, the drive means 67 continuously rotates the rotatable filter body. The driving state detecting means 68 detects the rotational displacement of the wavelength selecting element 66, which is a rotatable filter body, for example. The driving state detecting means 68 outputs BPF state data that indicate rotational displacement positions of the rotatable filter body.

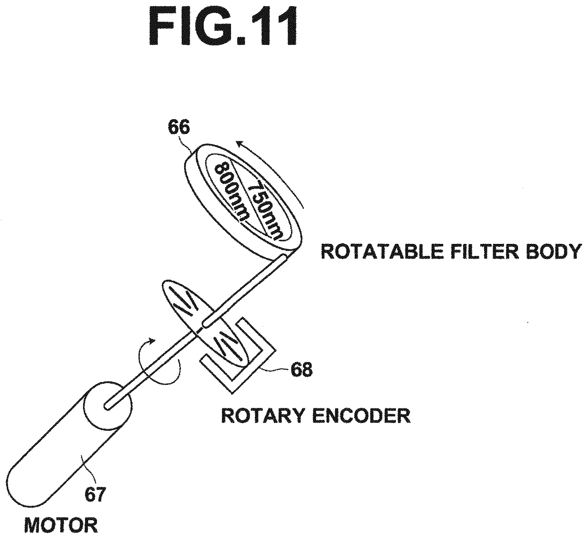

Figure 11 illustrates an example of the configurations of the wavelength selecting element 66, the drive means 67, and the driving state detecting means 68. In this example, the wavelength selecting element 66 is a rotatable filter body that includes two band pass filters, and the drive means is a servo motor. In addition, the driving state detecting means 68 is a rotary encoder. The wavelength selecting element 66 rotates according to rotation of an output shaft of the servo motor. Half of the rotatable filter body (rotational displacement positions from 0° to 180°, for example) is formed as the first band pass filter that transmits light having a wavelength of 750nm, and the other half of the rotatable filter body (rotational displacement positions from 180° to 360°, for example) is formed as the second band pass filter that transmits light having a wavelength of 800nm, for example. By rotating such a rotatable filter body, the first band pass filter and the second band pass filter can be alternately inserted into the optical path of the optical resonator at a switching speed corresponding to the rotating speed of the rotatable filter body.

The rotary encoder that constitutes the driving state detecting means 68 detects the rotational displacement of the wavelength selecting element 66, which is a rotatable filter body, with a rotatable plate having a slit mounted on the output shaft of the servo motor, and a transmissive type photo interrupter, and generates BPF state data. The driving state detecting means 68 outputs the BPF state data that represent rotational displacement positions of the rotatable filter body to the BPF control circuit 69.

Returning to Figure 10, the BPF control circuit 69 controls the drive means 67. The BPF control circuit 69 controls voltage which is supplied to the drive means 67 such that the amount of rotational displacement detected by the driving state detecting means 68 within a predetermined amount of time becomes an amount corresponding to a predetermined rotational speed of the rotatable filter body, for example. The BPF control circuit 69 monitors the BPF state data and controls the voltage supplied to the servo motor such that the amount of rotational displacement detected by the rotary encoder during a predetermined amount of time is maintained at an amount corresponding to the specified rotational speed, for example. The trigger control circuit 28 may be employed instead of the BPF control circuit 69 to monitor the BPF state data and control the drive means 67 such that the wavelength selecting element 66 is driven at a predetermined speed.

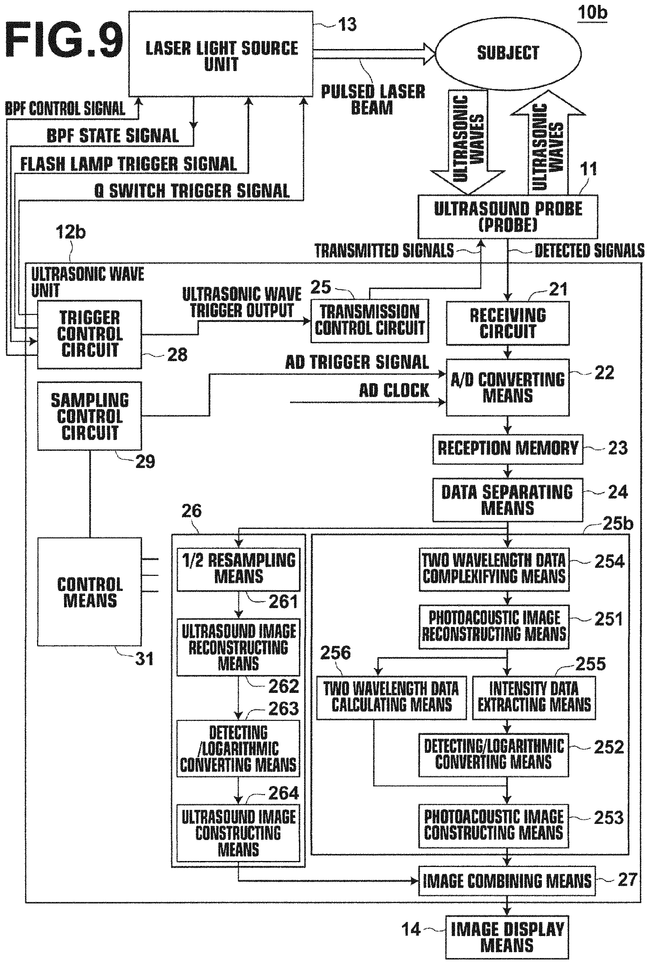

Returning to Figure 9, the control means 31 controls each of the components within the ultrasonic wave unit 12b. The trigger control circuit 28 controls the BPF control circuit 69 such that the band pass filters which are inserted into the optical path of the optical resonator within the laser unit 13 by the wavelength selecting element 66 are switched at a predetermined switching speed. The trigger control circuit 28 outputs BPF control signals that cause the rotatable filter body that constitutes the wavelength selecting element 66 to rotate continuously in a predetermined direction at a predetermined rotational speed, for example. The rotational speed of the rotatable filter body may be determined based on the number of wavelengths (the number of band pass filters) and the number of pulsed laser beams to be output by the laser unit 13 per unit time.

The trigger control circuit 28 outputs a flash lamp trigger signal to the laser unit 13 that causes the flash lamp 62 (Figure 10) to irradiate a pumping light beam onto the laser rod 61. The flash lamp 62 irradiates pumping light into the laser rod 61 in response to the flash lamp trigger signal. The trigger control circuit 28 outputs the flash lamp trigger signals at predetermined temporal intervals based on BPF state signals. For example, the trigger control circuit 28 outputs a flash lamp trigger signal when the BPF state data represents a position which is the driven position of the wavelength selecting element 66 at which the band pass filter corresponding to the wavelength of a pulsed laser beam to be output minus an amount of displacement that the wavelength selecting element will undergo during an amount of time necessary to pump the laser rod, to cause the pumping light beam to be irradiated onto the laser rod 61. The trigger control circuit 28 outputs the flash lamp trigger signals at periodically at predetermined temporal intervals, for example.

After outputting the flash lamp trigger signal, the trigger control circuit 28 outputs a Q switch trigger signal to the Q switch 65 of the laser unit 13. The trigger control circuit 28 outputs the Q switch trigger signal at a timing at which the band pass filter that transmits a wavelength corresponding to the wavelength of a pulsed laser beam to be output is inserted into the optical path of the optical resonator. For example, in the case that the wavelength selecting element 66 is constituted by a rotatable filter body, the trigger control circuit 28 outputs the Q switch trigger signal when the BPF state data indicates that a band pass filter corresponding to the wavelength of the pulsed laser beam to be output is inserted into the optical path of the optical resonator. The Q switch 65 changes the insertion loss within the optical resonator from high loss to low loss at high speed in response to the Q switch trigger signal, to output a pulsed laser beam from the output side mirror 64.

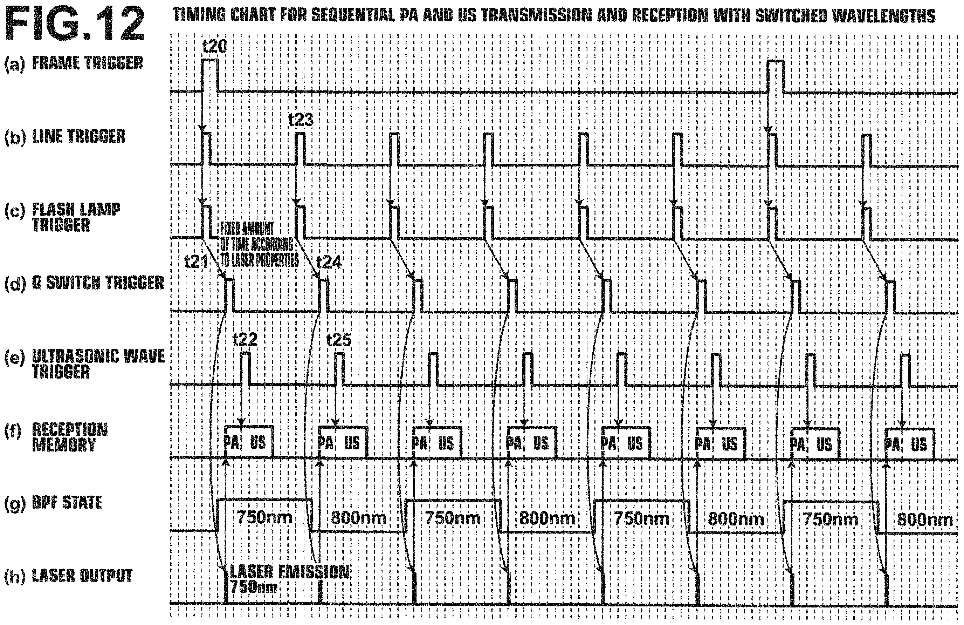

Figure 12 illustrates a timing chart of an exemplary operation. Here, the range in which the ultrasonic transducers of the probe 11 are arranged is divided into three regions, Area A, Area B, and Area C as illustrated in Figure 3, and photoacoustic signals and reflected acoustic signals are detected in order from Area A, Area B, and then Area C. The control means 31 generates a frame trigger signal (a). The frame rate is 10 frames/second, for example. In addition, the control means 31 generates a line trigger signal (b) having three pulses with respect to wavelengths of 750nm and 800nm, corresponding to each of Area A through Area C for a single frame. Because two light beams having two different wavelengths are to be irradiated onto a subject, there are six pulses in the line trigger for each frame. The areas are selected in order of Area A, Area B, then Area C. Light beams having wavelengths of 750nm and 800nm are alternately irradiated onto the subject.

The control means 31 outputs a pulse of the frame trigger signal and a first pulse of the line trigger signal at time t20. When the first pulse of the line trigger signal is output, the trigger control circuit 28 outputs a flash lamp trigger signal (c) to the laser unit 13. The laser unit 13 lights the flash lamp 32 and pumps the laser medium.

After outputting the flash lamp trigger signal, the trigger control circuit 28 outputs a Q switch trigger signal (d) at time t21. At this time, the band pass filter that transmits light having a wavelength of 750nm is inserted into the optical path of the optical resonator by the wavelength selecting element 66 (g). The amount of time from time t20 to time t21 may be set according to the properties of the laser. The laser unit 13 outputs a pulsed laser beam (h) having a wavelength of 750nm by the Q switch 33 being turned ON.

The receiving circuit 21 receives photoacoustic signals which are detected by the 64 ultrasonic transducers corresponding to Area A. The sampling control circuit 29 outputs a sampling trigger signal to the A/D converting means 22 synchronized with the timing of light irradiation. The A/D converting means 22 receives the sampling trigger signal, initiates sampling of the photoacoustic signals, and stores sampled data (first photoacoustic data) of the photoacoustic signals detected by the 64 ultrasonic transducers corresponding to Area A in the reception memory 23 (f).

The reception memory 23 stores therein sampled data of photoacoustic signals and reflected acoustic signals for a number of data points corresponding to the surface of the subject to a depth position of approximately 40mm. Specifically, the reception memory 23 stores sampled data of photoacoustic signals for 1024 points corresponding to approximately 40mm in the depth direction, and sampled data of reflected acoustic waves for 2048 points corresponding to approximately 40mm in the depth direction. In this case, the trigger control circuit 28 outputs an ultrasonic wave trigger signal (e) at time t22, which is the timing at which the A/D converting means 22 samples a 1024th piece of data from initiation of sampling. When the ultrasonic wave trigger signal is output, the probe 11 transmits ultrasonic waves from the 64 ultrasonic transducers that correspond to Area A, and detects reflected acoustic signals with the 64 ultrasonic transducers that correspond to Area A.

When switching from photoacoustic to ultrasonic waves, the A/D converting means 22 does not interrupt sampling of detected signals, and maintains a sampling state. The A/D converting means 22 stores sampled data of the reflected acoustic signals detected by the 64 ultrasonic transducers that correspond to Area A following the photoacoustic signals in the reception memory 23 (f). The reception memory 23 stores the first photoacoustic data and the reflected acoustic data for Area A as a series of data. Detection of photoacoustic signals with respect to light having a wavelength of 750nm and reflected acoustic signals for Area A is completed when 2048 pieces of sampled data of reflected acoustic signals are stored in the reception memory 23.

When detection at the wavelength of 750nm is completed, the control means 31 outputs a second pulse of the line trigger signal at time t23. In response to the output of the second pulse of the line trigger signal, the trigger control circuit 28 outputs a flash lamp trigger signal (c) to the laser unit 13. The laser unit 13 lights the flash lamp 32 and pumps the laser medium. After outputting the flash lamp trigger signal, the trigger control circuit 28 outputs a Q switch trigger signal (d) at time t24. At this time, the band pass filter that transmits light having a wavelength of 800nm is inserted into the optical path of the optical resonator by the wavelength selecting element 66 (g). The laser unit 13 outputs a pulsed laser beam (h) having a wavelength of 800nm by the Q switch 33 being turned ON.

The receiving circuit 21 receives photoacoustic signals which are detected by the 64 ultrasonic transducers corresponding to Area A. The sampling control circuit 29 outputs a sampling trigger signal to the A/D converting means 22 synchronized with the timing of light irradiation. The A/D converting means 22 receives the sampling trigger signal, initiates sampling of the photoacoustic signals, and stores sampled data (second photoacoustic data) of the photoacoustic signals detected by the 64 ultrasonic transducers corresponding to Area A in the reception memory 23 (f). The trigger control circuit 28 outputs an ultrasonic wave trigger signal (e) at time t22, which is the timing at which the A/D converting means 25 samples a 1024th piece of data from initiation of sampling.

The probe 11 transmits ultrasonic waves from the 64 ultrasonic transducers that correspond to Area A, and detects reflected acoustic signals with the 64 ultrasonic transducers that correspond to Area A. The A/D converting means 22 stores sampled data of the reflected acoustic signals detected by the 64 ultrasonic transducers that correspond to Area A following the photoacoustic signals in the reception memory 23 (f). The reception memory 23 stores the second photoacoustic data and the reflected acoustic data for Area A as a series of data. Detection of photoacoustic signals with respect to light having a wavelength of 800nm and reflected acoustic signals for Area A is completed when 2048 pieces of sampled data of reflected acoustic signals are stored in the reception memory 23.

When detection within Area A is completed, the operation moves to detection within Area B. Detection within Area B is performed in a manner similar to detection within Area A. That is, a Q switch trigger signal is output at a timing when the band pass filter that transmits light having a wavelength of 750nm is inserted into the optical path of the optical resonator by the wavelength selecting element 66, to irradiate light having a wavelength of 750nm onto the subject and to store first photoacoustic data in the reception memory 23. Ultrasonic waves are transmitted and received continuous to this operation, and reflected acoustic data are stored in the reception memory 23 continuous with the first photoacoustic data. Then, a Q switch trigger signal is output at a timing when the band pass filter that transmits light having a wavelength of 800nm is inserted into the optical path of the optical resonator by the wavelength selecting element 66, to irradiate light having a wavelength of 800nm onto the subject and to store second photoacoustic data in the reception memory 23. Ultrasonic waves are transmitted and received continuous to this operation, and reflected acoustic data are stored in the reception memory 23 continuous with the second photoacoustic data.

When detection within Area B is completed, the operation moves to detection within Area C. Detection within Area C is performed in a manner similar to detection within Area A.

Data necessary to generate photoacoustic images and an ultrasound image are collected in the reception memory 23 by detecting the photoacoustic signals for wavelengths of 750nm and 800 and the reflected acoustic signals in the three areas A through C. The data separating means 24 separates the photoacoustic signals and the reflected acoustic signals in the reception memory 23. The data separating means 24 reads out 1024 pieces of sampled data within a specific address range from among the 3072 pieces of sampled data for each element as first or second photoacoustic data, and reads out the remaining 1024 pieces of sampled data as reflected acoustic data to separate the photoacoustic data and the reflected acoustic data, for example. The photoacoustic image generating means 25 generates a photoacoustic image for each wavelength based on photoacoustic signals having 1024 data points for each element. Meanwhile, the ultrasound image generating means 26 employs the 1/2 resampling means 261 to resample the reflected acoustic signals having 2048 data points for each element into 1024 data points, and then generates an ultrasound image.

When detection (sampling) of the photoacoustic signals and the reflected acoustic signals for Area C is completed, the control means 31 outputs a pulse of a frame trigger signal (a) for a second frame. The operations following thereafter are the same as those following output of the first pulse of the frame trigger signal at time t20. The tomographic image generating apparatus 10 generates photoacoustic images and ultrasound images, by irradiating light having two wavelengths, detecting photoacoustic signals, transmitting ultrasonic waves, and detecting reflected acoustic signals within each area for each frame.

In the present embodiment, the laser unit 13 includes the wavelength selecting element 66, and the laser unit 13 is capable of irradiating a plurality of laser beams having wavelengths different from each other onto subjects. Laser beams having different wavelengths can be continuously switched and output by the laser unit 13b, by continuously driving the wavelength selecting element that includes two band pass filters that transmit different wavelengths, to continuously and selectively insert the two band pass filters into the optical path of the optical resonator, for example. Functional imaging that utilizes the fact that light absorption properties of light absorbers differ according to wavelengths is enabled by employing photoacoustic signals (photoacoustic data) obtained by irradiating pulsed laser beams having different wavelengths.

In the present embodiment, complex number data, in which one of the first photoacoustic data and the second photoacoustic data is designated as a real part and the other is designated as an imaginary part, are generated, and a reconstructed image is generated from the complex number data by the Fourier transform method. In such a case, only a single reconstruction operation is necessary, and reconstruction can be performed more efficiently compared to a case in which the first photoacoustic data and the second photoacoustic data are reconstructed separately. The advantageous effect that the amount of time required from initiation of obtainment of photoacoustic signals to completion of obtainment of reflected acoustic signals is shortened because the photoacoustic data and the reflected acoustic data are sampled as a series of data is the same as the first embodiment.

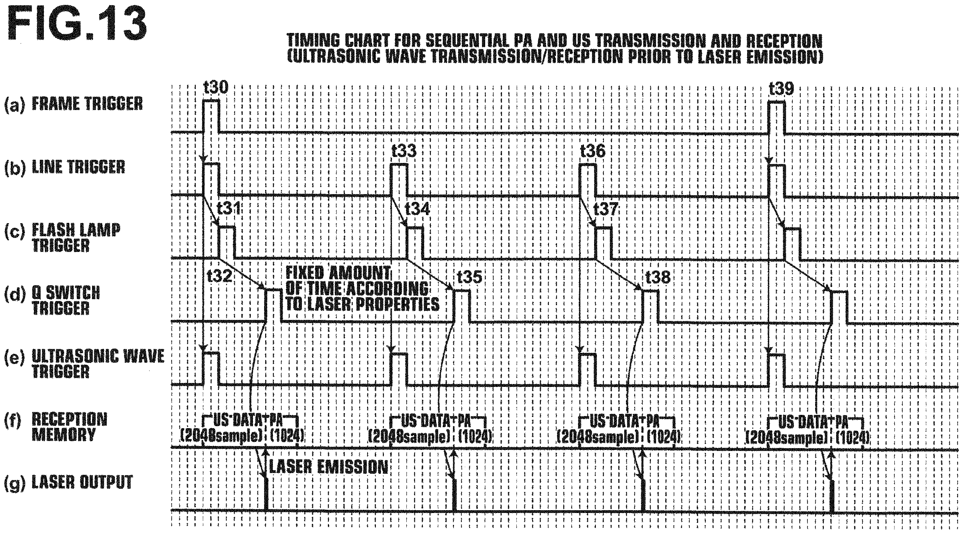

Note that in the embodiments described above, the photoacoustic signals were sampled first, and the reflected acoustic signals were sampled thereafter. Alternatively, reflected acoustic signals may be sampled first, and photoacoustic signals may be sampled thereafter. For example, the tomographic image generating apparatus 10 of the first embodiment illustrated in Figure 1 may transmit and receive ultrasonic waves first, then perform irradiation of light onto a subject and detection of photoacoustic signals thereafter. Figure 13 illustrates an exemplary operation in such a case. Here, pulses of three line trigger signals (b) corresponding to each of Areas A through C are output for each pulse of a frame trigger signal (a), in the same manner as in the example of Figure 5.

The control means 31 outputs a pulse of the frame trigger signal (a) and a first pulse of the line trigger signal (b) at time t30. When the first pulse of the line trigger signal is output, the trigger control circuit 28 outputs an ultrasonic wave trigger signal (e) to the transmission control circuit 30. When the ultrasonic wave trigger signal is output, the probe 11 transmits ultrasonic waves from the 64 ultrasonic transducers that correspond to Area A, and detects reflected acoustic signals with the 64 ultrasonic transducers that correspond to Area A.

The receiving circuit 21 receives the reflected acoustic signals detected by the 64 ultrasonic transducers that correspond to Area A. The sampling control circuit 29 outputs a sampling trigger signal to the A/D converting means 22 at a timing synchronized with transmission of the ultrasonic waves. The A/D converting means 22 receives the sampling trigger signal, initiates sampling of the reflected acoustic signals, and stores sampled data of the reflected acoustic signals detected by the 64 ultrasonic transducers that correspond to Area A in the reception memory 23 (f).

After outputting the ultrasonic wave trigger signal, the trigger control circuit 28 outputs a flash lamp trigger signal (c) to the laser unit 13 at time t31. The laser unit 13 lights the flash lamp 32 and pumps the laser medium. After outputting the flash lamp trigger signal, the trigger control circuit 28 outputs a Q switch trigger signal (d) at time t32. The amount of time from time t31 to time t32 may be set according to the properties of the laser. The laser unit 13 outputs a pulsed laser beam (g) by the Q switch 33 being turned ON.

The timing at which the laser unit 13 outputs the pulsed laser beam is the same as the timing at which sampling of the reflected acoustic signals is completed. The trigger control circuit 28 outputs the flash lamp trigger signal and the Q switch trigger signal such that the pulsed laser beam is irradiated onto the subject at a timing at which the A/D converting means samples a 2048th piece of data from initiation of sampling, for example.

After light is irradiated onto the subject, the receiving circuit 21 receives photoacoustic signals which are detected by the 64 ultrasonic transducers corresponding to Area A. When switching from ultrasonic waves to photoacoustic signals, the A/D converting means 22 does not interrupt sampling of detected signals, and maintains a sampling state. The A/D converting means 22 stores sampled data of the photoacoustic signals detected by the 64 ultrasonic transducers that correspond to Area A following the reflected acoustic signals in the reception memory 23 (f). The reception memory 23 stores the reflected acoustic signals and the photoacoustic signals for Area A as a series of data. Detection of reflected acoustic signals and photoacoustic signals for Area A is completed when 1024 pieces of sampled data of photoacoustic signals are stored in the reception memory 23.

When detection within Area A is completed, the control means 31 outputs a second pulse of the line trigger signal (b) at time t33. In response to the output of the second pulse of the line trigger signal, the trigger control circuit 28 outputs an ultrasonic wave trigger signal (e) to the transmission control circuit 30. When the ultrasonic wave trigger signal is output, the probe 11 transmits ultrasonic waves from the 64 ultrasonic transducers that correspond to Area B, and detects reflected acoustic signals with the 64 ultrasonic transducers that correspond to Area B.

The receiving circuit 21 receives the reflected acoustic signals detected by the 64 ultrasonic transducers that correspond to Area B. The sampling control circuit 29 outputs a sampling trigger signal to the A/D converting means 22 at a timing synchronized with transmission of the ultrasonic waves. The A/D converting means 22 receives the sampling trigger signal, initiates sampling of the reflected acoustic signals, and stores sampled data of the reflected acoustic signals detected by the 64 ultrasonic transducers that correspond to Area B in the reception memory 23 (f).

After outputting the ultrasonic wave trigger signal, the trigger control circuit 28 outputs a flash lamp trigger signal (c) to the laser unit 13 at time t34. The laser unit 13 lights the flash lamp 32 and pumps the laser medium. After outputting the flash lamp trigger signal, the trigger control circuit 28 outputs a Q switch trigger signal (d) at time t35, which is a timing at which the A/D converting means samples a 2048th piece of data from initiation of sampling. The laser unit 13 outputs a pulsed laser beam (g) by the Q switch 33 being turned ON.

After light is irradiated onto the subject, the receiving circuit 21 receives photoacoustic signals which are detected by the 64 ultrasonic transducers corresponding to Area B. The A/D converting means 22 stores sampled data of the photoacoustic signals detected by the 64 ultrasonic transducers that correspond to Area B following the reflected acoustic signals in the reception memory 23 (f). The reception memory 23 stores the reflected acoustic signals and the photoacoustic signals for Area B as a series of data. Detection of reflected acoustic signals and photoacoustic signals for Area B is completed when 1024 pieces of sampled data of photoacoustic signals are stored in the reception memory 23.

When detection within Area B is completed, the control means 31 outputs a second pulse of the line trigger signal (b) at time t36. In response to the output of the second pulse of the line trigger signal, the trigger control circuit 28 outputs an ultrasonic wave trigger signal (e) to the transmission control circuit 30. The probe 11 transmits ultrasonic waves from the 64 ultrasonic transducers that correspond to Area C, and detects reflected acoustic signals with the 64 ultrasonic transducers that correspond to Area C.

The receiving circuit 21 receives the reflected acoustic signals detected by the 64 ultrasonic transducers that correspond to Area C. The sampling control circuit 29 outputs a sampling trigger signal to the A/D converting means 22 at a timing synchronized with transmission of the ultrasonic waves. The A/D converting means 22 receives the sampling trigger signal, initiates sampling of the reflected acoustic signals, and stores sampled data of the reflected acoustic signals detected by the 64 ultrasonic transducers that correspond to Area C in the reception memory 23 (f).

After outputting the ultrasonic wave trigger signal, the trigger control circuit 28 outputs a flash lamp trigger signal (c) to the laser unit 13 at time t37. The laser unit 13 lights the flash lamp 32 and pumps the laser medium. After outputting the flash lamp trigger signal, the trigger control circuit 28 outputs a Q switch trigger signal (d) at time t38, which is a timing at which the A/D converting means samples a 2048th piece of data from initiation of sampling. The laser unit 13 outputs a pulsed laser beam (g) by the Q switch 33 being turned ON.

After light is irradiated onto the subject, the receiving circuit 21 receives photoacoustic signals which are detected by the 64 ultrasonic transducers corresponding to Area C. The A/D converting means 22 stores sampled data of the photoacoustic signals detected by the 64 ultrasonic transducers that correspond to Area C following the reflected acoustic signals in the reception memory 23 (f). The reception memory 23 stores the reflected acoustic signals and the photoacoustic signals for Area C as a series of data. Detection of reflected acoustic signals and photoacoustic signals for Area C is completed when 1024 pieces of sampled data of photoacoustic signals are stored in the reception memory 23.

Data necessary to generate a photoacoustic image and an ultrasound image are collected in the reception memory 23 by detecting the photoacoustic signals and the reflected acoustic signals in the three areas A through C. When detection (sampling) of the photoacoustic signals and the reflected acoustic signals for Area C is completed, the control means 31 outputs a pulse of a frame trigger signal (a) at time t39. The operations following thereafter are the same as those following output of the first pulse of the frame trigger signal at time t30. The same advantageous effects as those obtained by the first embodiment can be obtained by the operation described above. In the case that transmission and reception of ultrasonic waves are performed first in the second embodiment, the sampling rate may be controlled to be twice the sampling rate during detection of reflected acoustic signals synchronized with the timing at which light is irradiated.

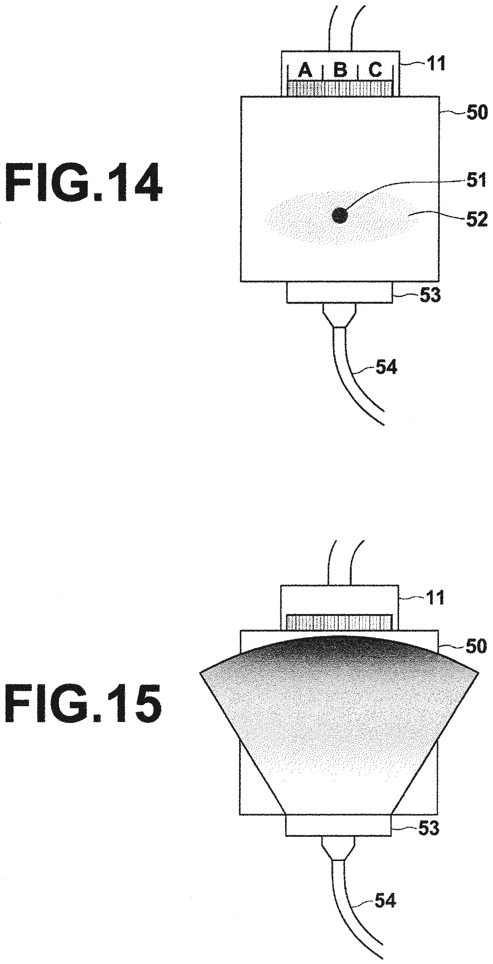

In addition, Figure 4A illustrates a case in which the probe 11 irradiates light. However, the location from which light is irradiated is not limited, and may be arbitrary. Figure 14 illustrates an example in which light is irradiated from a position facing the probe 11. The subject 50 has a light absorber 51 that generates photoacoustic waves due to irradiation of a pulsed laser beam, and a reflector 52 that reflects ultrasonic waves, directly under the probe 11. A light guiding plate 53 is provided as a light irradiating section at a position that faces the probe 11 with the subject 50 interposed therebetween. Light from the laser unit 13 is guided to the light guiding plate 53 using a light guiding means such as an optical fiber 54.

Figure 15 illustrates the manner in which light is irradiated. In this example, the light guided to the light guiding plate 53 using the optical fiber 54 is irradiated onto the subject from a position that faces the probe 11. The difference between this configuration and the example illustrated in Figure 4A is that in Figure 4A, light is irradiated from the same surface as an ultrasonic wave detecting surface, whereas in Figure 15, light is irradiated from a position that faces the ultrasonic wave detecting surface. Detection of photoacoustic signals and reflected acoustic signals may be performed in the same manner as that described for the first embodiment (Figure 4B through Figure 4D).

Note that the third embodiment was described as an example in which the first photoacoustic data and the second photoacoustic data were complexified. Alternatively, the first photoacoustic and the second photoacoustic data may be reconstructed separately without administering the complexifying operation. In addition, the reconstruction method is not limited to the Fourier transform method. Further, the second and third embodiments calculate the ratio between the first photoacoustic data and the second photoacoustic data by employing the phase data obtained by the complexifying operation. However, the same effects can be obtained by calculating the ratio using the intensity data of the first and second photoacoustic data. In addition, the intensity data may be generated based on signal intensities within a first reconstructed image and signal intensities within a second reconstructed image.

The number of pulsed laser beams having different wavelengths which are irradiated onto a subject when generating photoacoustic images is not limited to two. Three or more pulsed laser beams may be irradiated onto the subject, and photoacoustic images may be generated based on photoacoustic data corresponding to each wavelength. In this case, the two wavelength data calculating means 256 may generate the relationships among signal intensities of photoacoustic data corresponding to each wavelength as phase data. In addition, the intensity data extracting means 255 may generate a sum of signal intensities of photoacoustic data corresponding to each wavelength as intensity data.

The third embodiment was described mainly as a case in which the wavelength selecting element 66 is constituted by a rotatable filter body having two band pass filter regions. However, it is only necessary for the wavelength selecting element 66 to be that which can change the wavelength of light that oscillates within the optical resonator, and is not limited to a rotatable filter body. For example, the wavelength selecting element may be constituted by a rotatable body having a plurality of band pass filters provided on the circumference thereof. It is not necessary for the wavelength selecting element 66 to be a rotatable body. For example, a plurality of band pass filters may be arranged in a row. In this case, the wavelength selecting element 66 may be driven such that the plurality of band pass filters are cyclically inserted into the optical path of the optical resonator, or the wavelength selecting element 66 may be reciprocally driven such that the plurality of band pass filters arranged in a row traverse the optical path of the optical resonator. As a further alternative, a wavelength selecting element such as a birefringent filter may be employed instead of the band pass filters. In addition, when selecting between two wavelengths, if the gains of the wavelengths are different, long pass filters or short pass filters may be utilized instead of the band pass filters. For example, in the case that an alexandrite laser outputs laser beams having wavelengths of 800nm and 750nm, selection of each wavelength is possible by utilizing a combination of long pass filters for 800nm and 750nm, because the gain of the 750nm laser beam is greater.

Preferred embodiments of the present invention have been described above. However, the tomographic image generating apparatus and the photoacoustic image generating method are not limited to the above embodiments. Various changes and modifications to the configurations of the above embodiments are included in the scope of the present invention.

## Claims

1. A tomographic image generating apparatus (10), comprising:
a laser light source unit (13) adapted to output a laser beam to be irradiated onto a subject;
an ultrasonic wave transmitting section (11) adapted to transmit ultrasonic waves toward the subject;
an acoustic signal detecting section (11) adapted to detect photoacoustic signals generated within the subject due to irradiation of the laser beam onto the subject and to detect reflected acoustic signals of the ultrasonic waves transmitted into the subject;
a trigger control section (28) adapted to output light trigger signals that command laser beam output to the laser light source unit (13) and to output ultrasonic wave trigger signals that command transmission of ultrasonic waves to the ultrasonic wave transmitting section (11);
sampling means (22) adapted to sample the photoacoustic signals and the reflected acoustic signals and to store the sampled photoacoustic signals and the sampled reflected acoustic signals in a common memory (23) as a series of data;
sampling control means (29) adapted to output sampling trigger signals that command sampling initiation to the sampling means (22);
photoacoustic image generating means (25) adapted to generate photoacoustic images based on photoacoustic signals stored in the memory (23); and
ultrasound image generating means (26) adapted to generate ultrasound images based on reflected acoustic signals stored in the memory (23);
the trigger control section (28) is adapted to output one of a light trigger signal and a ultrasonic wave trigger signal, then outputting the other of a light trigger signal and a ultrasonic wave trigger signal in a state during which the sampling means (22) is continuing sampling; and
the sampling means (22) is adapted to continuously sample the photoacoustic signals and the reflected acoustic signals without interrupting a sampling operation.

2. A tomographic image generating apparatus (10) as defined in Claim 1, wherein:
the sampling control means (29) is adapted to output the sampling trigger signal at a timing having a predetermined temporal relationship with the timing at which the one of the light trigger signal and the ultrasonic wave trigger signal is output, and the trigger control section (28) is adapted to output the other of the light trigger signal and the ultrasonic wave trigger signal at a timing which is a predetermined time following output of the sampling trigger signal.

3. A tomographic image generating apparatus (10) as defined in either one of Claim 1 and Claim 2, wherein:
the sampling means (22) is adapted to sample the photoacoustic signals and the reflected acoustic signals at the same sampling rate; and
the ultrasound image generating means (26) comprises a 1/2 resampling means (261) that is adapted to resample the sampled reflected acoustic signals to 1/2, and to generate the ultrasound images based on the 1/2 resampled reflected acoustic signals.

4. A tomographic image generating apparatus (10) as defined in Claim 3, wherein:
the 1/2 resampling means (261) is adapted to compress the sampled reflected acoustic waves to 1/2 in the direction of a temporal axis.

5. A tomographic image generating apparatus (10) as defined in either one of Claim 1 and Claim 2, further comprising:
sampling rate control means (34) adapted to controll the sampling rate of the sampling means (22); and wherein:
the sampling rate control means (34) is adapted to control the sampling rate when the sampling means (22) samples the reflected acoustic signals to be half the sampling rate of the sampling rate when sampling the photoacoustic signals.

6. A tomographic image generating apparatus (10) as defined in Claim 5, wherein:
the sampling rate control means (34) controls the sampling rate synchronized with the ultrasonic wave trigger signal or with the light trigger signal.

7. A tomographic image generating apparatus (10) as defined in any one of Claims 1 through 6, wherein:
the trigger control section (28) is adapted to output the ultrasonic wave trigger signal following output of the light trigger signal.

8. A tomographic image generating apparatus (10) as defined in any one of Claims 1 through 6, wherein:
the trigger control section (28) is adapted to output the light trigger signal following output of the ultrasonic wave trigger signal.

9. A tomographic image generating apparatus (10) as defined in any one of Claims 1 through 8, wherein:
a range onto which ultrasonic waves are transmitted from the ultrasonic wave transmitting section (11) and a range in which the acoustic signal detecting section (11) detects the photoacoustic signals and the reflected acoustic signals are each divided into a plurality of regions; and
the trigger control section (28) is adapted to output the light trigger signals and the ultrasonic wave trigger signals and the sampling control means (29) outputs the sampling trigger signals for each of the divided regions.

10. A tomographic image generating apparatus (10) as defined in any one of Claims 1 through 9, further comprising:
data separating means (24) adapted to separate photoacoustic signals and reflected acoustic signals which are stored in the memory (23).

11. A tomographic image generating apparatus (10) as defined in any one of Claims 1 through 10, further comprising:
image combining means (27) for combining the photoacoustic images and the ultrasound images.

12. A tomographic image generating apparatus (10) as defined in any one of Claims 1 through 11, wherein:
the laser light source unit (13) is adapted to output a plurality of laser light beams having wavelengths different from each other.

13. A tomographic image generating apparatus (10) as defined in Claim 12, wherein:
the laser light source unit (13) comprises: a laser medium (61), pumping means (62) adapted to pump the laser medium (61), a pair of mirrors (63, 64) that constitute an optical resonator, and a wavelength selecting element (66) provided within the optical resonator.

14. A tomographic image generating apparatus (10) as defined in any one of Claims 1 through 13, wherein:
acoustic signal detecting elements of the acoustic signal detecting section (11) also function as ultrasonic transmission elements of the ultrasonic wave transmitting section (11).

15. A tomographic image generating method, comprising the steps of:
executing one of irradiation of a laser beam onto a subject and transmission of ultrasonic waves toward the subject; (step A3);
initiating sampling by a sampling means (22) matched with an irradiation timing of the laser beam or the transmission timing of the ultrasonic waves (step A5);
sampling one of photoacoustic signals generated within the subject due to irradiation of the laser beam onto the subject and reflected acoustic signals of the ultrasonic waves transmitted into the subject, with the sampling means (22), and storing one of the sampled photoacoustic signals and the reflected acoustic signals in a memory (23) (step A6) as a series of data;
executing the other of irradiation of a laser beam onto the subject and transmission of ultrasonic waves toward the subject while the sampling means (22) is continuing sampling (step A8);
sampling the other of the photoacoustic signals and the reflected acoustic signals with the sampling means (22) continuously with sampling of the one of the photoacoustic signals and the reflected acoustic waves (step A9) and storing the other of the photoacoustic signals and the reflected acoustic signals in the memory (23) (step A10); and
generating photoacoustic images and ultrasound images based on the photoacoustic signals and the reflected acoustic signals stored in the memory (23) (step A12, step A13).

## Patentansprüche

1. Tomographische Bilderzeugungsvorrichtung (10), umfassend.
eine Laserlichtquelleneinheit (13), ausgebildet zum Ausgeben eines Laserstrahls zum Aufstrahlen auf ein Objekt;
einen Ultraschallwellen-Sendeabschnitt (11), ausgebildet zum Senden von Ultraschallwellen in Richtung des Objekts;
einen Akustiksignal-Nachweisabschnitt (11), ausgebildet zum Nachweisen photoakustischer Signale, die in dem Objekt erzeugt werden aufgrund der Aufstrahlung des Laserstrahls auf das Objekt, und zum Nachweisen reflektierter Akustiksignale der in das Objekt gesendeten Ultraschallwellen;
einen Triggersteuerabschnitt (28), ausgebildet zum Ausgeben von Lichttriggersignalen, die eine Laserstrahlausgabe für die Laserlichtquelleneinheit (13) anweisen, und zum Ausgeben von Ultraschallwellen-Triggersignalen, die ein Senden von Ultraschallwellen für den Ultraschallwellen-Sendeabschnitt (11) anweisen;
eine Abtasteinrichtung (22), ausgebildet zum Abtasten der photoakustischen Signale und der reflektierten Akustiksignale, und zum Speichern der abgetasteten photoakustischen Signale und der abgetasteten reflektierten Akustiksignale in einem gemeinsamen Speicher (23) als eine Folge von Daten;
eine Abtaststeuereinrichtung (29), ausgebildet zum Ausgeben von Abtast-Triggersignalen, die die Abtasteinrichtung (22) anweisen, eine Abtastung einzuleiten;
eine photoakustische Bilderzeugungseinrichtung (25), ausgebildet zum Erzeugen photoakustischer Bilder basierend auf in dem Speicher (23) gespeicherten photoakustischen Signalen; und
eine Ultraschallbild-Erzeugungseinrichtung (26), ausgebildet zum Erzeugen von Ultraschallbildern basierend auf in dem Speicher (23) gespeicherten reflektierten Akustiksignalen;
wobei der Triggersteuerabschnitt (28) dazu ausgebildet ist, eines von einem Lichttriggersignal und einem Ultraschallwellen-Triggersignal auszugeben und dann das andere von dem Lichttriggersignal und dem Ultraschallwellen-Triggersignal in einem Zustand auszugeben, während dessen die Abtasteinrichtung (22) den Abtastvorgang fortsetzt; und
die Abtasteinrichtung (22) dazu ausgebildet ist, die photoakustischen Signale und die reflektierten akustischen Signale ohne Unterbrechung eines Abtastvorgangs kontinuierlich abzutasten.

2. Tomographische Bilderzeugungsvorrichtung (10) nach Anspruch 1, bei der
die Abtaststeuereinrichtung (29) dazu ausgebildet ist, das Abtasttriggersignal zu einer Zeit auszugeben, die eine vorbestimmte zeitliche Beziehung zu der Zeit hat, zu der das eine von dem Lichttriggersignal und dem Ultraschallwellen-Triggersignal ausgegeben wird, und der Triggersteuerabschnitt (28) dazu ausgebildet ist, das andere von dem Lichttriggersignal und dem Ultraschallwellen-Triggersignal zu einer Zeit auszugeben, bei der es sich um eine vorbestimmte Zeit im Anschluss an die Ausgabe des Abtasttriggersignals handelt.

3. Tomographische Bilderzeugungsvorrichtung (10) nach Anspruch 1 oder Anspruch 2, bei der:
die Abtasteinrichtung (22) dazu ausgebildet ist, die photoakustischen Signale und die reflektierten akustischen Signale mit der gleichen Abtastrate abzutasten; und
die Ultraschallbild-Erzeugungseinrichtung (26) eine 1/2-Neuabtasteinrichtung (261), ausgebildet zum Neuabtasten der abgetasteten reflektierten Akustiksignale zu 1/2 und zum Erzeugen der Ultraschallbilder basierend auf den 1/2-neuabgetasteten reflektierten Akustiksignalen aufweist.

4. Tomographische Bilderzeugungsvorrichtung (10) nach Anspruch 3, bei der
die 1/2-Neuabtasteinrichtung (261) dazu ausgebildet ist, die abgetasteten reflektierten akustischen Wellen auf 1/2 in Richtung einer Zeitachse zu komprimieren.

5. Tomographische Bilderzeugungsvorrichtung (10) nach Anspruch 1 oder Anspruch 2, weiterhin umfassend:
eine Abtastraten-Steuereinrichtung (34), ausgebildet zum Steuern der Abtastrate der Abtasteinrichtung (22); wobei
die Abtastraten-Steuereinrichtung (34) dazu ausgebildet ist, die Abtastrate so zusteuern, dass sie, wenn die Abtasteinrichtung (22) die reflektierten Akustiksignale abtastet, halb so groß ist wie die Abtastrate beim Abtasten der photoakustischen Signale.

6. Tomographische Bilderzeugungsvorrichtung (10) nach Anspruch 5, bei der
die Abtastraten-Steuereinrichtung (34) die Abtastrate synchronisiert mit dem Ultraschallwellen-Triggersignal oder mit dem Lichttriggersignal steuert.

7. Tomographische Bilderzeugungsvorrichtung (10) nach einem der Ansprüche 1 bis 6, bei der
der Triggersteuerabschnitt (28) dazu ausgebildet ist, das Ultraschallwellen-Triggersignal im Anschluss an die Ausgabe des Lichttriggersignals auszugeben.

8. Tomographische Bilderzeugungsvorrichtung (10) nach einem der Ansprüche 1 bis 6, bei der
der Triggersteuerabschnitt (28) dazu ausgebildet ist, das Lichttriggersignal im Anschluss an die Ausgabe des Ultraschallwellen-Triggersignals auszugeben.

9. Tomographische Bilderzeugungsvorrichtung (10) nach einem der Ansprüche 1 bis 8, bei der ein Bereich, auf den Ultraschallwellen von dem Ultraschallwellen-Sendeabschnitt (10) gesendet werden, und ein Bereich, in welchem der Akustiksignal-Nachweisabschnitt (11) die photoakustischen Signale und die reflektierten akustischen Signale nachweist, jeweils in mehrere Zonen unterteilt sind; und
der Triggersteuerabschnitt (28) dazu ausgebildet ist, die Lichttriggersignale und die Ultraschallwellen-Triggersignale auszugeben, und die Abtaststeuereinrichtung (29) die Abtasttriggersignale für jede der abgeteilten Zonen ausgibt.

10. Tomographische Bilderzeugungsvorrichtung (10) nach einem der Ansprüche 1 bis 9, weiterhin umfassend:
eine Datensepariereinrichtung (24), ausgebildet zum Separieren photoakustischer Signale und reflektierter akustischer Signale, die in dem Speicher (23) gespeichert sind.

11. Tomographische Bilderzeugungsvorrichtung (10) nach einem der Ansprüche 1 bis 10, weiterhin umfassend:
eine Bildkombiniereinrichtung (27) zum Kombinieren der photoakustischen Bilder und der Ultraschallbilder.

12. Tomographische Bilderzeugungsvorrichtung (10) nach einem der Ansprüche 1 bis 11, bei der
die Laserlichtquelleneinheit (13) dazu ausgebildet ist, mehrere Laserlichtstrahlen mit voneinander verschiedenen Wellenlängen auszugeben.

13. Tomographische Bilderzeugungsvorrichtung (10) nach Anspruch 12, bei der die Laserlichtquelleneinheit (13) aufweist: ein Lasermedium (61), eine Pumpeinrichtung (62), ausgebildet zum Pumpen des Lasermediums (61), ein Paar Spiegel (63, 64), die einen optischen Resonator bilden, und ein Wellenlängenselektionselement (66), das in dem optischen Resonator vorgesehen ist.

14. Tomographische Bilderzeugungsvorrichtung (10) nach einem der Ansprüche 1 bis 13, bei der
akustische Signalnachweiselemente des akustischen Signalnachweisabschnitts (11) auch als Ultraschall-Sendeelemente des Ultraschallwellen-Sendeabschnitts (11) fungieren.

15. Tomographisches Bilderzeugungsverfahren, umfassend die Schritte:
Ausführen einer Aufstrahlung eines Laserstrahls auf ein Objekt oder eines Sendens von Ultraschallwellen in Richtung des Objekts (Schritt A3);
Einleiten der Abtastung durch eine Abtasteinrichtung (22), angepasst an eine Aufstrahlzeit des Laserstrahls oder die Sendezeit der Ultraschallwellen (Schritt A5);
Abtasten von photoakustischen Signalen, die in dem Objekt aufgrund der Aufstrahlung des Laserstrahls auf das Objekt erzeugt werden, oder von reflektierten akustischen Signalen der in das Objekt gesendeten Ultraschallwellen, mit der Abtasteinrichtung (22), und Speichern der abgetasteten photoakustischen Signale bzw. der reflektierten akustischen Signale in einem Speicher (23) als eine Folge von Daten (Schritt A6);
Ausführen des Sendens von Ultraschallwellen in Richtung des Objekts bzw. des Aufstrahlens eines Laserstrahls auf das Objekt, während die Abtasteinrichtung (22) den Abtastvorgang fortsetzt (Schritt A8);
Abtasten der reflektierten akustischen Signale bzw. der photoakustischen Signale mit der Abtasteinrichtung (22) kontinuierlich bei der Abtastung der reflektierten akustischen Wellen bzw. der photoakustischen Signale (Schritt A9) und Speichern der photoakustischen Signale bzw. der reflektierten akustischen Signale in dem Speicher (23) (Schritt A10); und
Erzeugen photoakustischer Bilder und Ultraschallbilder basierend auf den photoakustischen Signalen bzw. den reflektierten akustischen Signalen, die in dem Speicher (23) gespeichert sind (Schritt A12, Schritt A13).

## Revendications

1. Appareil de génération d'images tomographiques (10), comprenant :
une unité de source de lumière laser (13) apte à produire un faisceau laser destiné à irradier un sujet ;
une section de transmission d'ondes ultrasoniques (11) apte à transmettre des ondes ultrasoniques vers le sujet ;
une section de détection de signaux acoustiques (11) apte à détecter des signaux photoacoustiques générés dans le sujet suite à l'irradiation du faisceau laser sur le sujet, et à détecter des signaux acoustiques réfléchis des ondes ultrasoniques transmises dans le sujet ;
une section de contrôle de déclenchement (28) apte à produire des signaux de déclenchement de lumière qui commandent la production de faisceau laser vers l'unité de source de lumière laser (13), et à produire des signaux de déclenchement d'ondes ultrasoniques qui commandent la transmission d'ondes ultrasoniques vers la section de transmission d'ondes ultrasoniques (11) ;
un moyen d'échantillonnage (22) apte à échantillonner les signaux photoacoustiques et les signaux acoustiques réfléchis, et à stocker les signaux photoacoustiques échantillonnés et les signaux acoustiques réfléchis échantillonnés dans une mémoire commune (23) sous la forme d'une série de données ;
un moyen de contrôle d'échantillonnage (29) apte à produire des signaux de déclenchement d'échantillonnage qui commandent une initialisation d'échantillonnage vers le moyen d'échantillonnage (22) ;
un moyen de génération d'images photoacoustiques (25) apte à générer des images photoacoustiques sur la base de signaux photoacoustiques stockés dans la mémoire (23), et
un moyen de génération d'images ultrasonores (26) apte à générer des images ultrasonores sur la base de signaux acoustiques réfléchis stockés dans la mémoire (23) ;
la section de contrôle de déclenchement (28) est apte à produire un signal parmi un signal de déclenchement de lumière et un signal de déclenchement d'ondes ultrasoniques, puis à produire l'autre signal parmi un signal de déclenchement de lumière et un signal de déclenchement d'ondes ultrasoniques dans un état durant lequel le moyen d'échantillonnage (22) continue d'échantillonner, et
le moyen d'échantillonnage (22) est apte à échantillonner en continu les signaux photoacoustiques et les signaux acoustiques réfléchis sans interrompre le fonctionnement d'échantillonnage.

2. Appareil de génération d'images tomographiques (10) selon la revendication 1, dans lequel :
le moyen de contrôle d'échantillonnage (29) est apte à produire le signal de déclenchement d'échantillonnage sur une temporisation présentant une relation temporelle prédéterminée avec la temporisation à laquelle le un signal parmi le signal de déclenchement de lumière et le signal de déclenchement d'ondes ultrasoniques est produit, et la section de contrôle de déclenchement (28) est apte à produire l'autre signal parmi le signal de déclenchement de lumière et le signal de déclenchement d'ondes ultrasoniques sur une temporisation qui est un temps prédéterminé suite à la production du signal de déclenchement d'échantillonnage.

3. Appareil de génération d'images tomographiques (10) selon la revendication 1 ou 2, dans lequel :
le moyen d'échantillonnage (22) est apte à échantillonner les signaux photoacoustiques et les signaux acoustiques réfléchis à la même fréquence d'échantillonnage, et
le moyen de génération d'images ultrasonores (26) comprend un moyen de rééchantillonnage 1/2 (261) apte à rééchantillonner les signaux acoustiques réfléchis échantillonnés à 1/2, et à générer les images ultrasonores sur la base des signaux acoustiques réfléchis rééchantillonnés à 1/2.

4. Appareil de génération d'images tomographiques (10) selon la revendication 3, dans lequel
le moyen de rééchantillonnage 1/2 (261) est apte à comprimer les ondes acoustiques réfléchies échantillonnées à 1/2 dans la direction d'un axe temporel.

5. Appareil de génération d'images tomographiques (10) selon la revendication 1 ou 2, comprenant en outre :
un moyen de contrôle de cadence d'échantillonnage (34) apte à contrôler la cadence d'échantillonnage du moyen d'échantillonnage (22), et dans lequel :
le moyen de contrôle de cadence d'échantillonnage (34) est apte à contrôler la cadence d'échantillonnage lorsque le moyen d'échantillonnage (22) échantillonne les signaux acoustiques réfléchis pour qu'elle soit égale à la moitié de la cadence d'échantillonnage de la cadence échantillonnage lors de l'échantillonnage des signaux photoacoustiques.

6. Appareil de génération d'images tomographiques (10) selon la revendication 5, dans lequel :
le moyen de contrôle de cadence d'échantillonnage (34) contrôle la cadence d'échantillonnage synchronisée avec le signal de déclenchement d'ondes ultrasoniques ou avec le signal de déclenchement de lumière.

7. Appareil de génération d'images tomographiques (10) selon l'une quelconque des revendications 1 à 6, dans lequel :
la section de contrôle de déclenchement (28) est apte à produire le signal de déclenchement d'ondes ultrasoniques suite à la production du signal de déclenchement de lumière.

8. Appareil de génération d'images tomographiques (10) selon l'une quelconque des revendications 1 à 6, dans lequel :
la section de contrôle de déclenchement (28) est apte à produire le signal de déclenchement de lumière suite à la production du signal de déclenchement d'ondes ultrasoniques.

9. Appareil de génération d'images tomographiques (10) selon l'une quelconque des revendications 1 à 8, dans lequel
un intervalle sur lequel des ondes ultrasoniques sont transmises à partir de la section de transmission d'ondes ultrasoniques (11) et un intervalle dans lequel la section de détection de signaux acoustiques (11) détecte les signaux photoacoustiques et les signaux acoustiques réfléchis sont chacun divisés en une pluralité de régions, et
la section de contrôle de déclenchement (28) est apte à produire les signaux de déclenchement de lumière et les signaux de déclenchement d'ondes ultrasoniques, et le moyen de contrôle d'échantillonnage (29) produit les signaux de déclenchement d'échantillonnage pour chacune des régions divisées.

10. Appareil de génération d'images tomographiques (10) selon l'une quelconque des revendications 1 à 9, comprenant en outre :
un moyen de séparation de données (24) apte à séparer des signaux photoacoustiques et des signaux acoustiques réfléchis qui sont stockés dans la mémoire (23).

11. Appareil de génération d'images tomographiques (10) selon l'une quelconque des revendications 1 à 10, comprenant en outre :
un moyen de combinaison d'images (27) destiné à combiner les images photoacoustiques et les images ultrasonores.

12. Appareil de génération d'images tomographiques (10) selon l'une quelconque des revendications 1 à 11, dans lequel :
l'unité de source de lumière laser (13) est apte à produire une pluralité de faisceaux de lumière laser présentant des longueurs d'onde différentes entre eux.

13. Appareil de génération d'images tomographiques (10) selon la revendication 12, dans lequel :
l'unité de source de lumière laser (13) comprend : un milieu laser (61), un moyen de pompage (62) apte à pomper le milieu laser (61), une paire de miroirs (63, 64) constituant un résonateur optique, et un élément de sélection de longueur d'onde (66) prévu dans le résonateur optique.

14. Appareil de génération d'images tomographiques (10) selon l'une quelconque des revendications 1 à 13, comprenant en outre :
des éléments de détection de signaux acoustiques de la section de détection de signaux acoustiques (11) fonctionnant également comme des éléments de transmission ultrasoniques de la section de transmission d'ondes ultrasoniques (11).

15. Procédé de génération d'images tomographiques, comprenant les étapes consistant à :
exécuter une étape parmi l'irradiation d'un faisceau laser sur un sujet et la transmission d'ondes ultrasoniques vers le sujet (étape A3) ;
initialiser l'échantillonnage par un moyen d'échantillonnage (22) en correspondance avec une temporisation d'irradiation du faisceau laser ou la temporisation de transmission des ondes ultrasoniques (étape A5) ;
échantillonner un signal parmi des signaux photoacoustiques générés dans le sujet suite à l'irradiation du faisceau laser sur le sujet et des signaux acoustiques réfléchis des ondes ultrasoniques transmises dans le sujet, avec le moyen d'échantillonnage (22), et stocker un signal parmi les signaux photoacoustiques échantillonnés et les signaux acoustiques réfléchis dans une mémoire commune (23) (étape A6) sous la forme d'une série de données ;
exécuter l'autre étape parmi l'irradiation d'un faisceau laser sur le sujet et la transmission d'ondes ultrasoniques vers le sujet tandis que le moyen d'échantillonnage (22) continue l'échantillonnage (étape A8) ;
échantillonner l'autre signal parmi les signaux photoacoustiques et les signaux acoustiques réfléchis avec le moyen d'échantillonnage (22) en continu avec l'échantillonnage du un signal parmi les signaux photoacoustiques et les signaux acoustiques réfléchis (étape (A9), et stocker l'autre signal parmi les signaux photoacoustiques et les signaux acoustiques réfléchis dans la mémoire (23) (étape A10), et
générer des images photoacoustiques et des images ultrasonores sur la base des signaux photoacoustiques et des signaux acoustiques réfléchis stockés dans la mémoire (23) (étape A 12, étape A13).
